# EUROPEAN PATENT APPLICATION

(11) **EP 4 714 955 A1**
(43) Date of publication of application: **25.03.2026**
(21) Application number: 24806671.4
(22) Date of filing: 17.05.2024
(51) Int. Cl.: C07D 489/08, C07D 221/28, C07D 217/20

(54) **VERSATILE INTERMEDIATE FOR OPIOID DERIVATIVES, AND PREPARATION METHOD THEREFOR AND USE THEREOF**

(30) Priority: 18.05.2023 CN 202310562472
(71) Applicant: Shaoxing Zejun Pharmaceuticals Co., Ltd, Shaoxing, Zhejiang 312369 (CN)
(72) Inventor: CAO, Zhen, Hangzhou Bay Economic And Technological Development Zone, Shangyu Distridt Shaoxing, Zhejiang 312369 (CN); ZHENG, Xinhua, Hangzhou Bay Economic And Technological Development Zone, Shangyu Distridt Shaoxing, Zhejiang 312369 (CN); SHAO, Weitao, Hangzhou Bay Economic And Technological Development Zone, Shangyu Distridt Shaoxing, Zhejiang 312369 (CN); XIONG, Kaiqin, Hangzhou Bay Economic And Technological Development Zone, Shangyu Distridt Shaoxing, Zhejiang 312369 (CN); LI, Juan, Hangzhou Bay Economic And Technological Development Zone, Shangyu Distridt Shaoxing, Zhejiang 312369 (CN); YE, Chuanzhen, Hangzhou Bay Economic And Technological Development Zone, Shangyu Distridt Shaoxing, Zhejiang 312369 (CN); ZHANG, Qi, Hangzhou Bay Economic And Technological Development Zone, Shangyu Distridt Shaoxing, Zhejiang 312369 (CN); HE, Xianbin, Hangzhou Bay Economic And Technological Development Zone, Shangyu Distridt Shaoxing, Zhejiang 312369 (CN); WU, Chunyi, Hangzhou Bay Economic And Technological Development Zone, Shangyu Distridt Shaoxing, Zhejiang 312369 (CN)
(74) Representative: Michalski Hüttermann & Partner Patentanwälte mbB
(86) International application number: PCT/CN2024/093980
(87) International publication number: WO 2024/235322

(57) **Abstract**

Disclosed in the present invention are a versatile intermediate for opioid derivatives, and a preparation method therefor and a use thereof. The present invention provides a compound as represented by formula 7. The compound as represented by formula 7 provided by the present invention can be used for synthesizing multiple opioid derivatives, and has one or more of the following advantages: cheap and easily available raw materials, simple synthesis steps, and a high product yield.

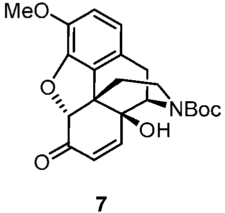

## Description

This application claims priority to Chinese Patent Application No. 2023105624725, filed on May 18, 2023. The aforementioned Chinese patent application is incorporated by reference in its entirety.

### TECHNICAL FIELD

The present disclosure relates to a universal intermediate for opioid derivatives, a preparation method thereof, and a use thereof.

### BACKGROUND

Naltrexone, naloxone, nalbuphine, oxymorphone, and oxycodone are all important opioid drugs widely used in postoperative analgesia and clinical practice. Among them, naltrexone and naloxone are opioid receptor antagonists, while nalbuphine, oxymorphone, and oxycodone are opioid receptor agonists.

Naltrexone is a pure opioid receptor antagonist that blocks µ-, δ-, and κ-opioid receptors. It significantly attenuates or completely blocks opioid receptors with a long duration of action. Approved by the FDA in 1984 as an oral medication, it is used to block dependence on opioids and alcohol.

Naloxone is a morphine receptor antagonist that functions by blocking exogenous opioid receptor agonists and endogenous morphine-like substances, thereby preventing or reversing central inhibitory effects, for example, respiratory depression induced by opioids, as well as exerting anti-shock effects. After intravenous injection, it acts rapidly and lasts for 1 to 4 hours. It is capable of reversing opioid overdose poisoning and persistent postoperative respiratory depression, and also used for the differential diagnosis of drug addicts.

Nalbuphine is an opioid receptor sedative-type psychotropic drug, with sedative effects similar to morphine and some respiratory depressant effects. It also exhibits a ceiling effect in its inhibition, has no cardiovascular side effects, and is generally used clinically for postoperative analgesia.

Oxycodone is a semisynthetic pure opioid receptor agonist, with pharmacological effects and mechanisms of action similar to morphine. It primarily exerts analgesic effects by activating opioid receptors in the central nervous system, providing moderate analgesic potency suitable for relieving moderate to severe pain. Additionally, it possesses anxiolytic and sedative properties.

Oxymorphone is an opioid receptor agonist with effects similar to morphine. It is commonly used clinically as an analgesic, providing ten times the analgesic potency of morphine but with a higher addictive potential than morphine. It is indicated for the relief of moderate to severe pain, obstetric analgesia, and adjunctive anesthesia.

They share structural similarity, primarily differing in the substituents at the 17-nitrogen atom. It is thus of significant importance to develop a versatile common intermediate for the divergent synthesis of a series of opioid drugs. Current synthetic routes for opioids are all semi-synthetic pathways based on extracts from poppy, for example, morphine, codeine, papaverine, and thebaine, which entail the following uncertainties: 1) Poppy cultivation is highly susceptible to climate impacts; 2) The extraction cost of raw materials is relatively high; 3) Poppy cultivation leads to soil quality degradation, etc. Therefore, the development of chemical synthesis processes serves as a vital supplement to the synthesis and production of opioids, particularly the creation of versatile common intermediates for opioid compounds, which holds even greater significance.

### SUMMARY

The technical problem to be solved by the present disclosure is the limited methods of synthesizing opioid derivatives in the prior art. To address this, the present disclosure provides a versatile intermediate for opioid derivatives, a preparation method thereof, and a use thereof. The intermediate provided by the present disclosure can be used to synthesize a variety of opioid derivatives and possesses one or more of the following advantages: low-cost and readily available raw materials, simplified synthesis steps, and high product yield.

The present disclosure addresses the above technical problem through the following solution.

The present disclosure provides a preparation method of a compound of formula A8, comprising the following steps S11, S12, and S13:
S11: performing a hydrogenation reaction as shown below on a compound of formula 7 with hydrogen in a solvent in the presence of a catalyst to obtain a compound of formula 8-1; 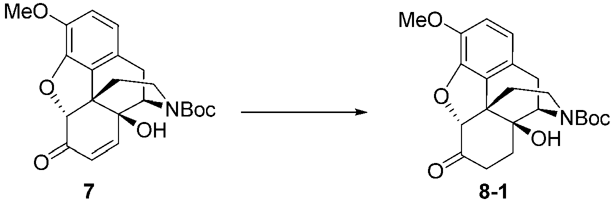
S12: performing a deprotection reaction as shown below on the compound of formula 8-1 in a solvent in the presence of a deprotecting reagent to obtain a compound of formula 8-2; 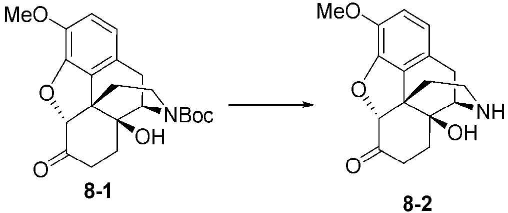
S13: performing a substitution reaction as shown below on the compound of formula 8-2 with a compound of formula SM1 in a solvent in the presence of a base to obtain a compound of formula A8. 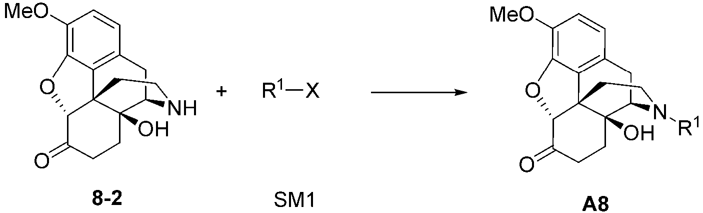
wherein X is halogen and R¹ is C₁₋₄ alkyl or C₁₋₄ alkyl substituted with one or more R¹⁻¹; R¹⁻¹ is 3- to 6-membered cycloalkyl or C₂₋₄ alkenyl.

In step S11, the conditions, types of reagents, and reagent dosages for the hydrogenation reaction may be conventional for such reactions in the art, with the following being preferred in the present disclosure.

In step S11, the solvent may be an alcohol solvent, for example, methanol and/or ethanol, for another example, methanol.

In step S11, the molar volume ratio of the compound of formula 7 to the solvent may be (0.1 to 0.3) mol:1 L, for example, 0.13 mol:1 L.

In step S11, the catalyst may be Raney nickel and/or a palladium catalyst, for example, a palladium catalyst, for another example, 10% palladium on carbon, where "%" represents the mass percentage of palladium relative to the total mass of palladium and carbon.

In step S11, the mass ratio of the catalyst to the compound of formula 7 may be (0.01 to 1):1, for example, (0.05 to 0.5): 1, for another example, 0.1:1.

In step S11, the pressure of the hydrogenation reaction may be 1 to 10 atm, for example, 1 to 3 atm, for another example, 2 atm.

In step S11, the temperature of the hydrogenation reaction may be 10 to 45°C, for example, 20 to 30°C, for another example, 30°C.

In step S11, the progress of the hydrogenation reaction may be monitored using conventional methods in the art (for example, TLC or LCMS), and the reaction endpoint is generally determined when the compound of formula 7 has disappeared or no longer reacts. The duration of the hydrogenation reaction may be 1 to 10 hours, for example, 3 to 6 hours, for another example, 4 hours.

In step S11, the following work-up operations may be comprised following completion of the hydrogenation reaction, which are conventional work-up operations for such a reaction, for example, filtration and concentration.

In step S11, the next reaction step may be performed without a purification step following completion of the hydrogenation reaction.

In step S12, the conditions, types of reagents, and reagent dosages for the deprotection reaction may be conventional for such a reaction in the art, with the following being preferred in the present disclosure.

In step S12, the solvent may be an alcohol solvent, for example, methanol and/or ethanol, for another example, methanol.

In step S12, the molar volume ratio of the compound of formula 8-1 to the solvent may be (0.01 to 1) mol:1 L, for example, 0.05 mol:1 L.

In step S12, the deprotecting reagent may be an acid, for example, an inorganic acid, for another example, HCl.

The acid may be a solution of acid, for example, a 4 mol/L solution of HCl in methanol.

In step S12, the molar ratio of the acid to the compound of formula 8-1 may be (1 to 100): 1, for example, (30 to 60):1, for another example, 43:1.

In step S12, the temperature of the deprotection reaction may be 0 to 20°C, for example, 10°C.

In step S12, the progress of the deprotection reaction may be monitored using conventional methods in the art (for example, TLC or LCMS), and the reaction endpoint is generally determined when the compound of formula 8-1 has disappeared or no longer reacts. The duration of the deprotection reaction may be 0.5 to 3 hours, for example, 1.5 hours.

In step S12, the following work-up operations may be comprised following completion of the deprotection reaction, which are conventional work-up operations for such a reaction, for example, concentration.

In step S12, the next reaction step may be performed without a purification step following completion of the deprotection reaction.

In one embodiment, in X, the halogen may be F, Cl, Br, or I, for example, Br.

In one embodiment, in said R¹, the C₁₋₄ alkyl in said C₁₋₄ alkyl and said C₁₋₄ alkyl substituted with one or more R¹⁻¹ may independently be methyl, ethyl, n-propyl, isopropyl, tert-butyl, n-butyl, or isobutyl, for example, methyl.

In one embodiment, in said R¹, the C₁₋₄ alkyl substituted with one or more R¹⁻¹ is C₁₋₄ alkyl substituted with one R¹⁻¹.

In one embodiment, in said R¹⁻¹, the 3- to 6-membered cycloalkyl may be cyclopropyl, cyclobutyl, cyclopentyl, or cyclohexyl, for example, cyclopropyl or cyclobutyl.

In one embodiment, in said R¹⁻¹, the C₂₋₄ alkenyl may be vinyl, propenyl, or butenyl, for example, vinyl.

In one embodiment, R¹ may be methyl, 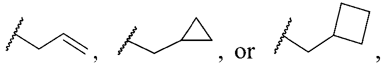 for example,

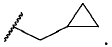

In one embodiment, X is Br.

In one embodiment, in step S13, the compound of formula SM1 is 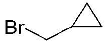 and the compound of formula A8 is

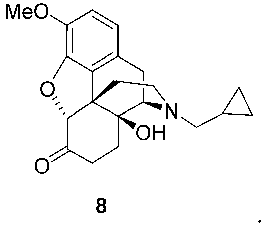

In step S13, the conditions, types of reagents, and reagent dosages for the substitution reaction may be conventional for such reactions in the art, with the following being preferred in the present disclosure.

In step S13, the solvent may be a mixed solvent of an organic solvent and water.

The organic solvent may be a pyrrolidone organic solvent, for example, N-methylpyrrolidone.

The volume ratio of the organic solvent to water may be (1 to 10):1, for example, (4 to 6):1, for another example, 5:1.

In step S13, the molar volume ratio of the compound of formula 8-2 to the solvent may be (0.01 to 0.3) mol:1 L, for example, 0.19 mol:1 L.

In step S13, the base may be an organic base, for example, an organic amine, for another example, triethylamine.

In step S13, the molar ratio of the base to the compound of formula 8-2 may be (1 to 10):1, for example, (4 to 6):1, for another example, 5:1.

In step S13, the molar ratio of the compound of formula SM1 to the compound of formula 8-2 may be (1 to 3):1, for example, (1 to 1.5): 1, for another example, 1.3:1.

In step S13, the substitution reaction may be performed under the atmosphere of an inert gas, which may be a conventional protective gas in the art, for example, nitrogen and/or argon, for another example, nitrogen.

In step S13, the temperature of the substitution reaction may be 60 to 90°C, for example, 70°C.

In step S13, the progress of the substitution reaction may be monitored using conventional methods in the art (for example, TLC or LCMS), and the reaction endpoint is generally determined when the compound of formula 8-2 has disappeared or no longer reacts. The duration of the substitution reaction may be 1 to 6 hours, for example, 4 hours.

In step S13, the following work-up operations may be comprised following completion of the substitution reaction, which are conventional work-up operations for such a reaction, for example, one or more of extraction, drying, concentration, and purification. A solvent for said extraction may be dichloromethane. A desiccant for said drying may be anhydrous sodium sulfate. Said concentration may be distillation under reduced pressure. Said purification may be column chromatography. An eluent for said column chromatography may be a mixed solvent of dichloromethane and methanol, for example, with the volume ratio of dichloromethane to methanol of 50:1.

The preparation method of the compound of formula A8 further comprises a preparation method of the compound of formula 7, specifically comprising the following step S10: performing an oxidation reaction as shown below on a compound of formula 6 in a solvent in the presence of an oxidant to obtain the compound of formula 7,

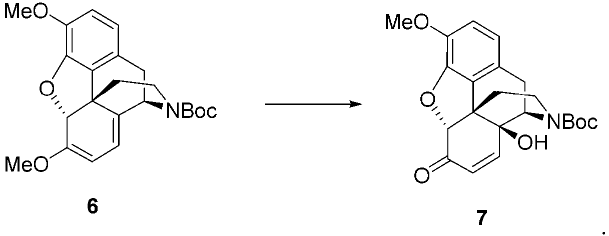

In step S10, the conditions, types of reagents, and reagent dosages for the oxidation reaction may be conventional for such reactions in the art, with the following being preferred in the present disclosure.

In step S10, the solvent may be one or more of an organic acid solvent, a chlorinated alkane solvent, and water, for example, a mixed solvent of an organic acid solvent, a chlorinated alkane solvent, and water.

The chlorinated alkane solvent may be one or more of dichloromethane, 1,2-dichloroethane, and chloroform, for example, dichloromethane.

The organic acid solvent may be formic acid and/or acetic acid, for example, acetic acid.

The ratio of the mixed solvent is not specifically limited, provided that it ensures good solubility and does not affect the reaction. In the present disclosure, the preferred ratio of the mixed solvent is as follows.

In the mixed solvent, the volume ratio of the chlorinated alkane solvent to water may be (1 to 10):1, for example, 7:1.

In the mixed solvent, the volume ratio of the organic acid solvent to water may be (1 to 10):1, for example, 2:1.

In step S10, the molar volume ratio of the compound of formula 6 to the solvent may be (0.01 to 1) mol:1 L, for example, (0.01 to 0.3) mol:1 mL, for another example, 0.13 mol:1 L.

The oxidant may be one or more selected from hydrogen peroxide, meta-chloroperoxybenzoic acid, and magnesium monoperoxyphthalate hexahydrate, for example, magnesium monoperoxyphthalate hexahydrate (MMPP).

In step S10, the molar ratio of the oxidant to the compound of formula 6 may be (1 to 5):1, for example, (1.1 to 2):1, for another example, 1.5:1.

In step S10, the temperature of the oxidation reaction may be -20°C to 25°C, for example, 0°C to 20°C, for another example, 15°C.

In step S10, the oxidation reaction may further comprise the step of mixing the compound of formula 6 with the solvent, and adding the oxidant.

The oxidant may be added at -5 to 5°C, for example, at 0°C.

In step S10, the progress of the oxidation reaction may be monitored using conventional methods in the art (for example, TLC or LCMS), and the reaction endpoint is generally determined when the compound of formula 6 has disappeared or no longer reacts. The duration of the oxidation reaction may be 1 to 5 hours, for example, 3 hours.

In step S10, the following work-up operations may be comprised following completion of the oxidation reaction, which may be conventional work-up operations for such a reaction, for example, one or more of quenching, extraction, drying, concentration, and purification. A solvent for said quenching may be an aqueous solution of sodium thiosulfate. A solvent for said extraction may be dichloromethane. A desiccant for said drying may be anhydrous sodium sulfate. Said concentration may be distillation under reduced pressure. Said purification may be column chromatography, where an eluent for said column chromatography may be a mixed solvent of dichloromethane and ethyl acetate, for example, with the volume ratio of dichloromethane to ethyl acetate of 4:1.

In the preparation method of the compound of formula A8, the preparation method of the compound of formula 7 further comprises a preparation method of the compound of formula 6, specifically comprising the following step S9: performing a cyclization reaction as shown below on a compound of formula 6-2 in a solvent in the presence of a cyclization agent to obtain the compound of formula 6,

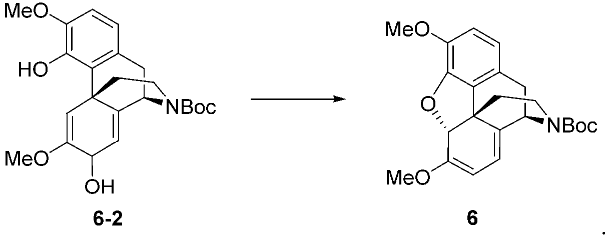

In step S9, the conditions, types of reagents, and reagent dosages for the cyclization reaction may be conventional for such reactions in the art, with the following being preferred in the present disclosure.

In step S9, the cyclization reaction may be performed under the atmosphere of an inert gas. The inert gas may be a conventional protective gas in the art, for example, nitrogen and/or argon, for another example, nitrogen.

In step S9, the solvent may be a chlorinated alkane solvent. The chlorinated alkane solvent may be one or more of dichloromethane, 1,2-dichloroethane, and chloroform, for example, dichloromethane.

In step S9, the molar volume ratio of the compound of formula 6-2 to the solvent may be (0.1 to 2) mol: 1 L, for example, 0.9 mol: 1 L.

In step S9, the cyclization agent may be one or more of N,N-dimethylformamide dimethyl acetal (DMFDMA), p-toluic acid, sodium methoxide, and sodium ethoxide, for example, N,N-dimethylformamide dimethyl acetal.

In step S9, the molar ratio of the cyclization agent to the compound of formula 6-2 may be (1 to 5):1, for example, (1 to 3):1, for another example, 1.5:1.

In step S9, the temperature of the cyclization reaction may be 20 to 35°C, for example, 25°C.

In step S9, the progress of the cyclization reaction may be monitored using conventional methods in the art (for example, TLC or LCMS), and the reaction endpoint is generally determined when the compound of formula 6-2 has disappeared or no longer reacts. The duration of the cyclization reaction may be 1 to 5 hours, for example, 3 hours.

In step S9, the following work-up operations may be comprised following completion of the cyclization reaction, which may be conventional work-up operations for such a reaction, for example, one or more of extraction, drying, concentration, and purification. A solvent for said extraction may be a mixed solvent of petroleum ether and ethyl acetate, for example, with a volume ratio of petroleum ether to ethyl acetate of 6:1. A desiccant for said drying may be anhydrous sodium sulfate. Said concentration may be distillation under reduced pressure. Said purification may be crystallization. A solvent for said crystallization may be a mixed solvent of methanol and water, for example, in a volume ratio of methanol to water of 4:1. The temperature of said crystallization may be -20 to 0°C, for example, -10°C.

In the preparation method of the compound of formula A8, the preparation method of the compound of formula 6 further comprises a preparation method of the compound of formula 6-2, specifically comprising the following step S8: performing a reduction reaction as shown below on a compound of formula 6-1 in a solvent in the presence of a reducing agent to obtain the compound of formula 6-2,

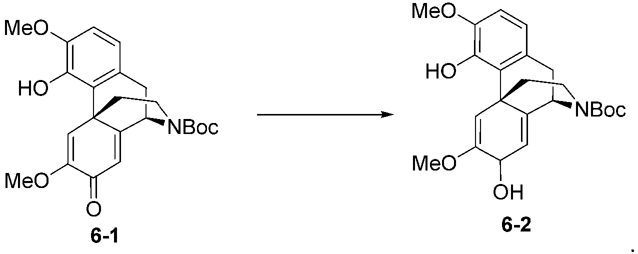

In step S8, the conditions, types of reagents, and reagent dosages for the reduction reaction may be conventional for such reactions in the art, with the following being preferred in the present disclosure.

In step S8, the reduction reaction may be performed under the atmosphere of an inert gas. The inert gas may be a conventional protective gas in the art, for example, nitrogen and/or argon, for another example, nitrogen.

In step S8, the solvent may be an ether solvent, for example, tetrahydrofuran.

In step S8, the molar volume ratio of the compound of formula 6-1 to the solvent may be (0.1 to 2) mol:1 L, for example, 0.22 mol:1 L.

In step S8, the reducing agent may be one or more selected from alkali metal borohydrides, lithium aluminum hydride, and diisobutylaluminum hydride, for example, diisobutylaluminum hydride, for another example, a solution of diisobutylaluminum hydride in tetrahydrofuran.

In step S8, the molar ratio of the reducing agent to the compound of formula 6-1 may be (1 to 5):1, for example, (1 to 3):1, for another example, 2:1.

In step S8, the temperature of the reduction reaction may be -5 to 35°C, for example, 10 to 30°C, for another example, 15°C.

In step S8, the progress of the reduction reaction may be monitored using conventional methods in the art (for example, TLC or LCMS), and the reaction endpoint is generally determined when the compound of formula 6-1 has disappeared or no longer reacts. The duration of the reduction reaction may be 0.1 to 2 hours, for example, 0.5 hours.

Step S8 may further comprise the following step of mixing the compound of formula 6-1 with the solvent, followed by adding the reducing agent to carry out the reduction reaction.

A reducing agent may be added at -25 to 5°C, for example, at -20 °C or 5 °C.

In step S8, the following work-up operations may be comprised following completion of the reduction reaction, which may be conventional work-up operations for such a reaction, for example, one or more of quenching, extraction, drying, and concentration. A solvent for said quenching may be an aqueous solution of sodium hydroxide, for example, an aqueous solution of sodium hydroxide with a molar concentration of 4 N. A solvent for said extraction may be dichloromethane. A desiccant for said drying may be anhydrous sodium sulfate. Said concentration may be distillation under reduced pressure.

In the preparation method of the compound of formula A8, the preparation method of the compound of formula 6-2 further comprises a preparation method of the compound of formula 6-1, specifically comprising the following step S7: performing a debenzylation reaction as shown below on a compound of formula 5 in a solvent in the presence of a hydrogen source and a palladium catalyst to obtain the compound of formula 6-1,

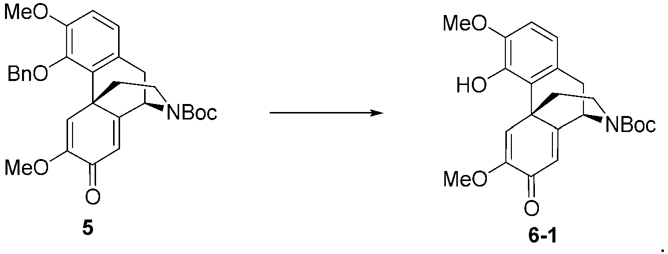

In step S7, the conditions, types of reagents, and reagent dosages for the debenzylation reaction may be conventional for such a reaction in the art, with the following being preferred in the present disclosure.

In step S7, the debenzylation reaction may be performed under the atmosphere of an inert gas. The inert gas may be a conventional protective gas in the art, for example, nitrogen and/or argon, for another example, nitrogen.

In step S7, the solvent may be an ether solvent or an amide solvent, for example, an amide solvent, for another example, N,N-dimethylacetamide.

In step S7, the molar volume ratio of the compound of formula 5 to the solvent may be (0.1 to 1) mol:1 L, for example, 0.4 mol:1 L.

In step S7, the hydrogen source may be hydrogen and/or sodium hydride, for example, sodium hydride.

In step S7, the molar ratio of the hydrogen source to the compound of formula 5 may be (1 to 2):1, for example, 1.5:1.

In step S7, the palladium catalyst may be one or more selected from dry palladium carbon, palladium hydroxide, palladium chloride, and palladium acetate, for example, palladium chloride.

In step S7, the molar ratio of the palladium catalyst to the compound of formula 5 may be (0.01 to 0.5):1, for example, 0.05:1.

In step S7, the temperature of the debenzylation reaction may be 10 to 30°C, for example, 25°C.

In step S7, the progress of the debenzylation reaction may be monitored using conventional methods in the art (for example, TLC or LCMS), and the reaction endpoint is generally determined when the compound of formula 5 has disappeared or no longer reacts. The duration of the debenzylation reaction may be 1 to 20 hours, for example, 10 hours.

In step S7, the following work-up operations may be comprised following completion of the debenzylation reaction, which may be conventional work-up operations for such a reaction, for example, one or more of quenching, extraction, drying, and concentration. A solvent for said quenching may be water. A solvent for said extraction may be ethyl acetate. A desiccant for said drying may be anhydrous sodium sulfate. Said concentration may be distillation under reduced pressure.

In the preparation method of the compound of formula A8, the preparation method of the compound of formula 6-1 further comprises a preparation method of the compound of formula 5, specifically comprising the following step S6: performing a dearomative cyclization reaction as shown below on a compound of formula 4 in a solvent under the action of a palladium catalyst, a phosphine ligand, and a base to obtain the compound of formula 5,

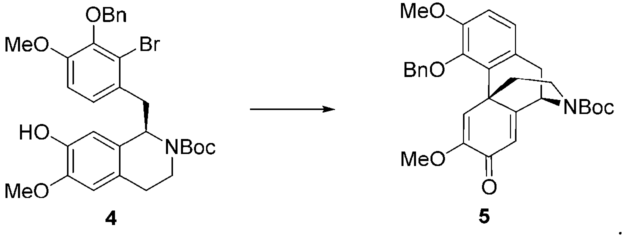

In step S6, the conditions, types of reagents, and reagent dosages for the dearomative cyclization reaction may be conventional for such reactions in the art, with the following being preferred in the present disclosure.

In step S6, the dearomative cyclization reaction may be performed under the atmosphere of an inert gas. The inert gas may be a conventional protective gas in the art, for example, nitrogen and/or argon, for another example, nitrogen.

In step S6, the solvent may be a sulfoxide solvent and/or an amide solvent, for example, an amide solvent. The amide solvent may be one or more of N-pyrrolidone, N,N-dimethylformamide (DMF), and N,N-dimethylacetamide (DMA), for another example, N,N-dimethylacetamide.

In step S6, the molar volume ratio of the compound of formula 4 to the solvent may be (0.01 to 1) mol:1 L, for example, 0.29 mol:1 L.

In step S6, the palladium catalyst may be one or more of palladium chloride, bis(acetonitrile)palladium dichloride, palladium trifluoromethanesulfonate, and palladium acetate, for example, palladium chloride.

In step S6, the molar ratio of the palladium catalyst to the compound of formula 4 may be (0.01 to 0.5): 1, for example, 0.04:1.

In step S6, the phosphine ligand may be one or more of tri-tert-butylphosphine, tricyclohexylphosphine, di(1-adamantyl)-n-butylphosphine, diadamantylisopropylphosphine, and diadamantylpropylphosphine, for example, di(1-adamantyl)-n-butylphosphine.

In step S6, the molar ratio of the phosphine ligand to the compound of formula 4 may be (0.01 to 0.5): 1, for example, 0.06:1.

In step S6, the base may be an alkali metal carbonate, for example, potassium carbonate or potassium phosphate.

In step S6, the molar ratio of the base to the compound of formula 4 may be (1 to 5):1, for example, 2:1.

In step S6, the temperature of the dearomative cyclization reaction may be 100°C to 180°C, for example, 140°C.

In step S6, the progress of the dearomative reaction may be monitored using conventional methods in the art (for example, TLC or LCMS), and the endpoint is generally determined when the compound of formula 4 has disappeared or no longer reacts. The duration of the dearomative reaction may be 3 to 10 hours, for example, 6 hours.

In step S6, the following work-up operations may be comprised following completion of the dearomative reaction, which are conventional work-up operations for such a reaction in the art, for example, one or more of filtration, concentration, drying, and purification. Said concentration may be distillation under reduced pressure. Said purification may be column chromatography, where an eluent for said column chromatography may be a mixed solvent of dichloromethane and methanol, for example, with a volume ratio of dichloromethane to methanol of (150 to 100):1.

In the preparation method of the compound of formula A8, the preparation method of the compound of formula 5 further comprises a preparation method of the compound of formula 4, specifically comprising the following step S5: performing a reaction as shown below on a compound of formula 4-3' with di-tert-butyl dicarbonate in a solvent to obtain the compound of formula 4,

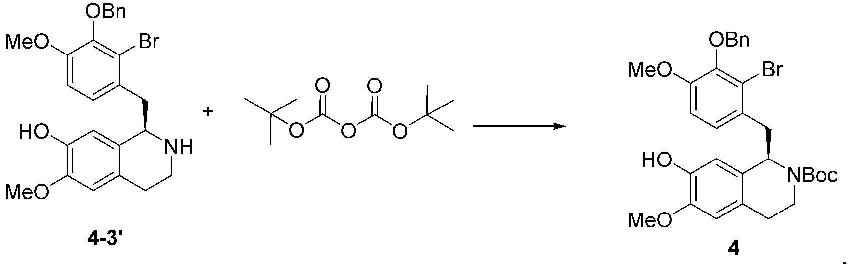

In step S5, the conditions, types of reagents, and reagent dosages for the reaction may be conventional for such reactions in the art, with the following being preferred in the present disclosure.

In step S5, the solvent may be a chlorinated alkane solvent, which may be one or more of dichloromethane, 1,2-dichloroethane, and chloroform, for example, dichloromethane.

In step S5, the molar volume ratio of the compound of formula 4-3' to the solvent may be (0.01 to 1) mol:1 L, for example, 0.09 mol:1 L.

In step S5, the molar ratio of di-tert-butyl dicarbonate to the compound of formula 4-3' may be (1 to 3):1, for example, 1.08:1.

In step S5, the temperature of the reaction may be 10 to 30°C, for example, 25°C.

In step S5, the progress of the reaction may be monitored using conventional methods in the art (for example, TLC or LC-MS), and the reaction endpoint is generally determined when the compound of formula 4-3' has disappeared or no longer reacts. The duration of the reaction may be 1 to 5 hours, for example, 3 hours.

In step S5, the following work-up operations may be comprised following completion of the reaction, which are conventional work-up operations for such a reaction in the art, for example, one or more of concentration, crystallization, filtration, and drying. Said concentration may be distillation under reduced pressure. A solvent for said crystallization may be methanol. Said drying may be oven drying.

In the preparation method of the compound of formula A8, the preparation method of the compound of formula 4 further comprises a preparation method of the compound of formula 4-3', specifically comprising the following step S4': performing a reaction as shown below on a compound of formula 4-3 with a base in a solvent to obtain the compound of formula 4-3',

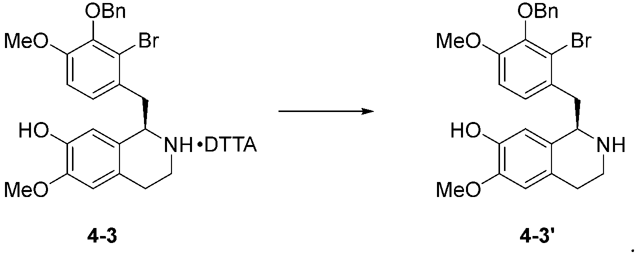

In step S4', the conditions, types of reagents, and reagent dosages for the reaction may be conventional for such reactions in the art, with the following being preferred in the present disclosure.

In step S4', the solvent may be a chlorinated hydrocarbon solvent. The chlorinated alkane solvent may be one or more of dichloromethane, 1,2-dichloroethane, and chloroform, for example, dichloromethane.

In step S4', the molar volume ratio of the compound of formula 4-3 to the solvent may be (0.01 to 1) mol:1 L, for example, 0.13 mol:1 L.

In step S4', the base may be an inorganic base, for example, potassium bicarbonate, for example, an aqueous solution of potassium bicarbonate.

In step S4', the molar ratio of the base to the compound of formula 4-3 may be (1 to 10):1, for example, (4 to 6):1, for another example, 5:1.

In step S4', the temperature of the reaction may be 20 to 30°C, for example, 25°C.

In step S4', the progress of the reaction may be monitored using conventional methods in the art (for example, TLC or LC-MS), and the reaction endpoint is generally determined when the compound of formula 4-3 has disappeared or no longer reacts. The duration of the reaction may be 0.3 to 12 hours, for example, 1 hour.

In step S4', after completion of the reaction, the following work-up step may be comprised: extraction. A solvent for said extraction may be dichloromethane.

In the preparation method of the compound of formula A8, the preparation method of the compound of formula 4-3' further comprises a preparation method of the compound of formula 4-3, specifically comprising the following step S4: performing a reaction as shown below on a compound of formula 4-2 with D-(+)-di-p-toluoyl tartaric acid (DTTA) in a solvent to obtain the compound of formula 4-3,

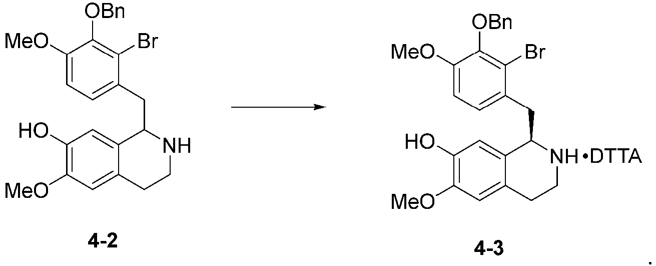

In step S4, the reaction may be performed in an open system, which may be an air system.

In step S4, the solvent may be an alcohol solvent, for example, one or more selected from methanol, ethanol, and isopropanol, for another example, methanol.

In step S4, the molar volume ratio of the compound of formula 4-2 to the solvent may be (0.1 to 1) mol:1 L, for example, 0.62 mol:1 L or 0.6 mol:1 L.

In step S4, the molar ratio of D-(+)-di-p-toluoyl tartaric acid to the compound of formula 4-2 may be (1 to 3):1, for example, (0.9 to 1.5):1, for another example, 1.05:1 or 1.07:1.

In step S4, the temperature of the reaction may be 50 to 100°C, for example, 70°C to 100°C, for another example, 85°C.

In step S4, the progress of the reaction may be monitored using conventional methods in the art (for example, TLC or LC-MS), and the reaction endpoint is generally determined when the compound of formula 4-2 has disappeared or no longer reacts. The duration of the reaction may be 3 to 12 hours, for example, 8 hours.

Step S4 may further comprise the following step: mixing the compound of formula 4-2 with the solvent, and subsequently adding D-(+)-di-p-toluoyl tartaric acid.

In step S4, after completion of the reaction, the following work-up step may be comprised: filtration.

In the preparation method of the compound of formula A8, the preparation method of the compound of formula 4-3 further comprises a preparation method of the compound of formula 4-2, specifically comprising the following step S3: performing a deprotection reaction of carbamate groups as shown below on a compound of formula 4-1 in a solvent in the presence of an organic base to obtain the compound of formula 4-2,

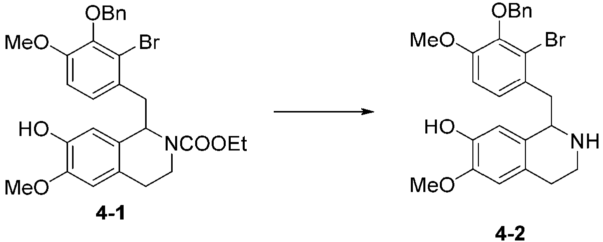

The organic base is potassium alkoxide and/or sodium alkoxide.

In step S3, the solvent may be an ether solvent and/or a chlorinated hydrocarbon solvent, for example, an ether solvent.

The ether solvent may be one or more of tetrahydrofuran, 1,4-dioxane, diethyl ether, and methyl tert-butyl ether, for example, tetrahydrofuran.

In step S3, the molar volume ratio of the compound of formula 4-1 to the solvent may be (0.01 to 3) mol:1 L, for example, (0.1-1) mol:1 L, for another example, 0.31 mol:1 L or 0.35 mol:1 L.

In step S3, the potassium alkoxide may be potassium methoxide, potassium ethoxide, or potassium tert-butoxide, for example, potassium tert-butoxide. The sodium alkoxide may be sodium methoxide, sodium ethoxide, or sodium tert-butoxide.

In step S3, the reaction system may further comprise an inorganic base. The inorganic base may be potassium hydroxide and/or sodium hydroxide, for example, potassium hydroxide.

The molar ratio of the organic base to the inorganic base may be (0.5 to 2):1, for example, 1:1.

In step S3, the molar ratio of the organic base to the compound of formula 4-1 may be (0.05 to 5):1, for example, (1 to 3):1, for another example, 2:1 or 1.77:1.

In step S3, the temperature of the deprotection reaction of carbamate groups may be 60 to 80°C, for example, 70°C.

Step S3 may further comprise the following step: mixing the compound of formula 4-1 with the solvent, and subsequently adding the organic base.

In step S3, the progress of the deprotection reaction of carbamate groups may be monitored using conventional methods in the art (for example, TLC or LCMS), and the endpoint is generally determined when the compound of formula 4-1 has disappeared or no longer reacts. The duration of the deprotection reaction of carbamate groups may be 5 to 24 hours, for example, 8 hours.

In step S3, the following work-up operations may be comprised following completion of the deprotection reaction of carbamate groups, which includes one or more of quenching, extraction, drying, and concentration. A solvent for said quenching may be water. A solvent for said extraction may be dichloromethane. Said concentration may be concentration under reduced pressure.

In the preparation method of the compound of formula A8, the preparation method of the compound of formula 4-2 further comprises a preparation method of the compound of formula 4-1, specifically comprising the following step S2: performing a reaction as shown below on a compound of formula 3' in a solvent in the presence of a base to obtain the compound of formula 4-1',

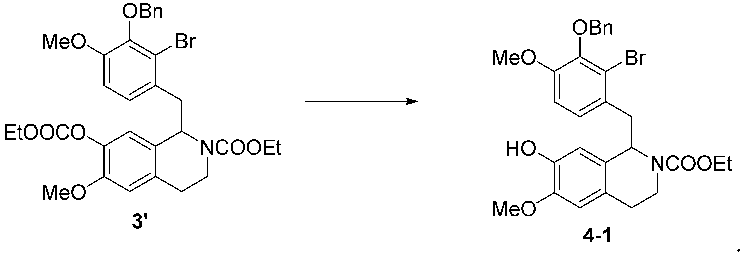

In step S2, the conditions, types of reagents, and reagent dosages for the reaction may be conventional for such reactions in the art, with the following being preferred in the present disclosure.

In step S2, the solvent may be an alcohol solvent, for example, methanol and/or ethanol, for another example, ethanol.

In step S2, the molar volume ratio of the compound of formula 3 to the solvent may be (0.1 to 1) mol:1 L, for example, 0.62 mol:1 L.

In step S2, the base may be an inorganic base, for example, potassium hydroxide.

In step S2, the molar ratio of the base to the compound of formula 3 may be (1 to 3):1, for example, 2:1.

In step S2, the temperature of the reaction may be 30°C to 70°C, for example, 50°C.

In step S2, the progress of the reaction may be monitored using conventional methods in the art (for example, TLC or LCMS), and the endpoint is generally determined when the compound of formula 3 has disappeared or no longer reacts. The duration of the reaction may be 1 to 10 hours, for example, 3 hours.

In step S2, the following work-up operations may be comprised following completion of the reaction, which may be conventional work-up operations for such a reaction, for example, one or more of quenching, slurrying, and drying. A solvent for said quenching may be water. Said drying may be oven drying. A solvent for said slurrying may be methanol.

In the preparation method of the compound of formula A8, the preparation method of the compound of formula 4-1 further comprises a preparation method of the compound of formula 3', specifically comprising the following step S2': performing a reaction as shown below on a compound of formula 3 with benzyl bromide in a solvent in the presence of a base to obtain the compound of formula 3',

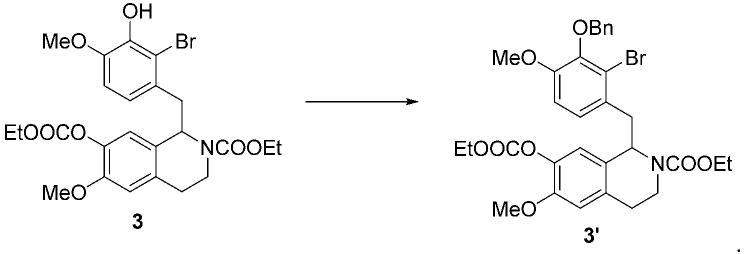

In step S2', the conditions, types of reagents, and reagent dosages for the reaction may be conventional for such reactions in the art, with the following being preferred in the present disclosure.

In step S2', the reaction may be performed under the atmosphere of an inert gas. The inert gas may be a conventional protective gas in the art, for example, nitrogen and/or argon, for another example, nitrogen.

In step S2', the solvent may be an amide solvent, for example, N,N-dimethylformamide (DMF) and/or N,N-dimethylacetamide (DMA), for another example, N,N-dimethylformamide.

In step S2', the molar volume ratio of the compound of formula 3 to the solvent may be (0.1 to 1) mol:1 L, for example, 0.62 mol:1 L.

In step S2', the base may be an alkali metal carbonate, for example, potassium carbonate.

In step S2', the molar ratio of the base to the compound of formula 3 may be (1 to 3):1, for example, 2:1.

In step S2', the molar ratio of the benzyl bromide to the compound of formula 3 may be (1 to 3):1, for example, 2:1.

In step S2', the temperature of the reaction may be 10°C to 30°C, for example, 25°C.

In step S2', the progress of the reaction may be monitored using conventional methods in the art (for example, TLC or LCMS), and the endpoint is generally determined when the compound of formula 3 has disappeared or no longer reacts. The duration of the reaction may be 3 to 10 hours, for example, 6 hours.

In step S2', the following work-up operations may be comprised following completion of the reaction, which may be conventional work-up operations for such a reaction, for example, crystallization. A solvent for said crystallization may be water.

In the preparation method of the compound of formula A8, the preparation method of the compound of formula 3' further comprises a preparation method of the compound of formula 3, specifically comprising the following step S1: performing a condensation cyclization reaction as shown below on a compound of formula 1 and a compound of formula 2 in a solvent in the presence of a acid to obtain the compound of formula 3,

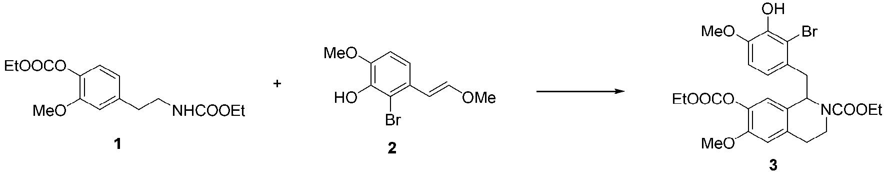

The acid is one or more of methanesulfonic acid, trifluoroacetic acid, p-toluenesulfonic acid, and p-toluenesulfonic acid monohydrate.

In step S1, the condensation cyclization reaction may be performed in an open system. The open system may be an air system.

In step S1, the solvent is a conventional solvent for such a reaction in the art, which may be a chlorinated alkane and/or an amide, for example, a chlorinated alkane organic solvent.

The chlorinated alkane solvent may be one or more of dichloromethane, 1,2-dichloroethane, and chloroform, for example, dichloromethane.

In step S1, the molar volume ratio of the compound of formula 1 to the solvent may be (0.1 to 5) mol:1 L, for example, (0.1 to 2) mol:1 L, for example, 0.78 mol:1 L.

In step S1, the molar ratio of the acid to the compound of formula 1 may be (1 to 5):1, for example, (1 to 3):1, for another example, 2:1.

In step S1, the acid may be p-toluenesulfonic acid and/or p-toluenesulfonic acid monohydrate, for example, p-toluenesulfonic acid monohydrate.

In step S1, the molar ratio of the compound of formula 2 to the compound of formula 1 may be (1 to 1.5):1, for example, 1.4:1.

In step S1, the temperature of the condensation cyclization reaction may be 0 to 30°C, for example, 25°C.

In step S1, the progress of the condensation cyclization reaction may be monitored using conventional methods in the art (for example, TLC or LCMS), and the endpoint is generally determined when the compound of formula 1 has disappeared or no longer reacts. The duration of the reaction may be 2 to 24 hours, for example, 16 hours.

In step S1, the following work-up operations may be comprised following completion of the reaction, which may be conventional work-up operations for such a reaction, for example, one or more of quenching, drying, concentration, and purification. A solvent for said quenching may be water. A desiccant for said drying may be anhydrous sodium sulfate. Said concentration may be distillation under reduced pressure. Said purification may be recrystallization, and a solvent for said recrystallization may be a mixed solvent of ethyl acetate and petroleum ether, for example, in a volume ratio of ethyl acetate to petroleum ether of 2:1.

In the preparation method of the compound of formula A8, the preparation method of the compound of formula 3 further comprises a preparation method of the compound of formula 1, specifically comprising the following step S1-1: performing a reaction as shown below on a compound of formula 1-2 with ethyl chloroformate in a solvent to obtain the compound of formula 1,

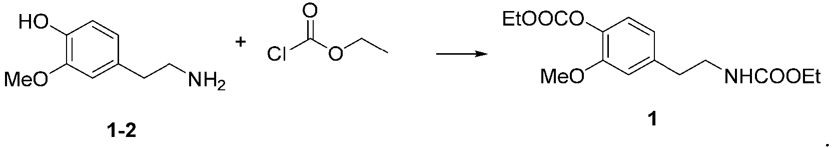

In step S1-1, the solvent may be a conventional solvent for such a reaction in the art, for example, an ether solvent, for another example, tetrahydrofuran.

In step S1-1, the molar volume ratio of the compound of formula 1-2 to the solvent may be (0.1 to 5) mol:1 L, for example, (0.1 to 1) mol:1 L, for another example, 0.1 mol:1 L.

In step S1-1, the molar ratio of ethyl chloroformate to the compound of formula 1-2 may be (1 to 5):1, for example, (1 to 3):1, for another example, 2.2:1.

In step S1-1, the temperature of the reaction may be 10 to 30°C, for example, 25°C.

In step S1-1, the progress of the reaction may be monitored using conventional methods in the art (for example, TLC or LCMS), and the endpoint is generally determined when the compound of formula 1 has disappeared or no longer reacts. The duration of the reaction may be 1 to 30 hours, for example, 16 hours.

In step S1-1, the following work-up operations may be comprised following completion of the reaction, which may be conventional work-up operations for such a reaction, for example, one or more of quenching, extraction, drying, and concentration. A solvent for said quenching may be water. A desiccant for said drying may be anhydrous sodium sulfate. Said concentration may be distillation under reduced pressure. Said purification may be recrystallization, and a solvent for said extraction may be ethyl acetate.

The present disclosure provides a preparation method of a compound of formula A9, comprising the following step S14: performing a demethylation reaction as shown below on the compound of formula A8 in a solvent in the presence of a demethylating agent to obtain a compound of formula A9.

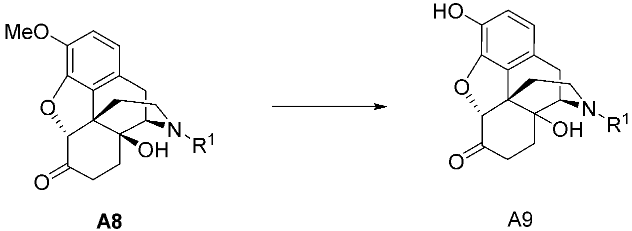

The preparation method of the compound of formula A9 further comprises a preparation method of the compound of formula A8, specifically comprising the following steps S11, S12, and S13:
S11: performing a hydrogenation reaction as shown below on a compound of formula 7 with hydrogen in a solvent in the presence of a catalyst to obtain a compound of formula 8-1; 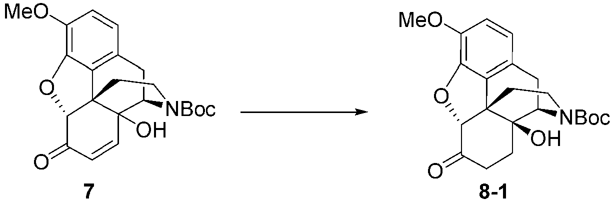
S12: performing a deprotection reaction as shown below on the compound of formula 8-1 in a solvent in the presence of a deprotecting reagent to obtain a compound of formula 8-2; 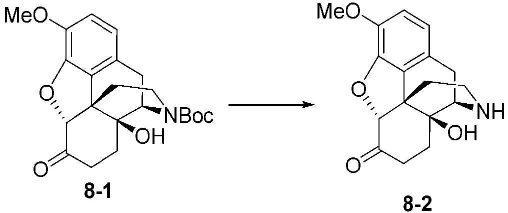
S13: performing a substitution reaction as shown below on the compound of formula 8-2 with a compound of formula SM1 in a solvent in the presence of a base to obtain a compound of formula A8. 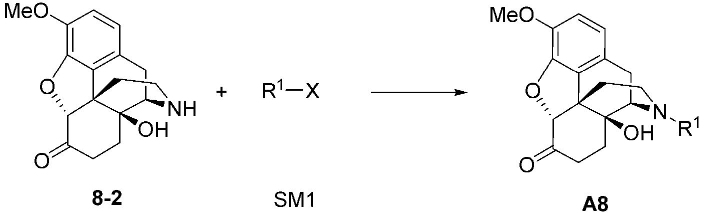
wherein X and R¹ are as defined in any one of the embodiments of the present disclosure.

In one embodiment, in step S14, the compound of formula A8 is 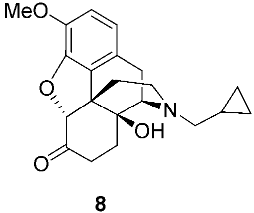 and the compound of formula A9 is

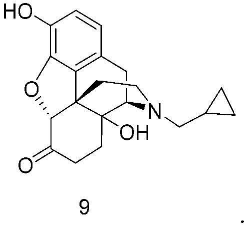

In step S14, the reaction conditions, types of reagents, and reagent dosages for the reaction may be conventional for such reactions in the art, with the following being preferred in the present disclosure.

In step S14, the solvent may be a chlorinated alkane, which may be one or more of dichloromethane, 1,2-dichloroethane, and chloroform, for example, dichloromethane.

In step S14, the molar volume ratio of the compound of formula A8 to the solvent may be (0.01 to 0.1) mol:1 L, for example, 0.045 mol:1 L.

In step S14, the demethylation reaction may be performed under the atmosphere of an inert gas. The inert gas may be a conventional protective gas in the art, for example, nitrogen and/or argon, for another example, nitrogen.

In step S14, the demethylating agent may be boron trichloride and/or boron tribromide, for example, boron tribromide.

In step S14, the molar ratio of the demethylating agent to the compound of formula A8 may be (5 to 10):1, for example, (6 to 8):1, for another example, 7:1.

In step S14, the temperature of the demethylation reaction may be -20 to 25°C, for example, 10°C.

In step S14, the progress of the demethylation reaction may be monitored using conventional methods in the art (for example, TLC or LCMS), and the reaction endpoint is generally determined when the compound of formula 8 has disappeared or no longer reacts. The duration of the demethylation reaction may be 1 to 5 hours, for example, 2 hours.

In step S14, the following work-up operations may be comprised following completion of the demethylation reaction, which are conventional work-up operations for such a reaction, for example, one or more of quenching, pH adjustment, extraction, drying, concentration, and purification. A solvent for said quenching may be a saturated aqueous solution of ammonium chloride. A solvent for said pH adjustment may be a saturated aqueous solution of 10% NaHCO₃. A solvent for said extraction may be dichloromethane. A desiccant for said drying may be anhydrous sodium sulfate. Said concentration may be distillation under reduced pressure. Said purification may be column chromatography. An eluent for said column chromatography may be a mixed solvent of dichloromethane and methanol, for example, with a volume ratio of dichloromethane to methanol of 50:1.

The conditions and operations for the preparation method of the compound of formula A8 may be as described in any one of the embodiments of the present disclosure.

The present disclosure provides a preparation method of the compound of formula 4-3', comprising the following step S4': performing a reaction as shown below on the compound of formula 4-3 with a base in a solvent to obtain the compound of formula 4-3',

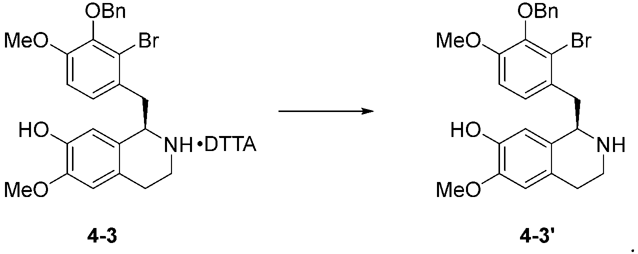

The conditions and operations for the preparation method of the compound of formula 4-3' may be as described in any one of the embodiments of the present disclosure.

The preparation method of the compound of formula 4-3' may further comprise a preparation method of the compound of formula 4-3, specifically comprising the following step S4: performing a reaction as shown below on a compound of formula 4-2 with D-(+)-di-p-toluoyl tartaric acid (DTTA) in a solvent to obtain the compound of formula 4-3,

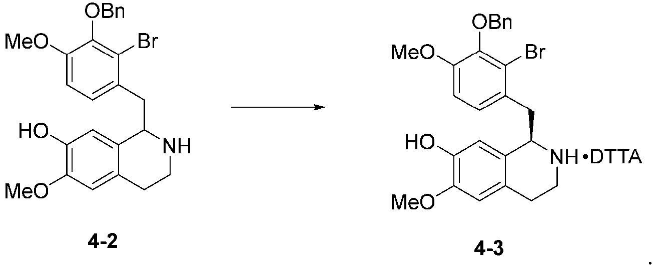

The conditions and operations for the preparation method of the compound of formula 4-3 may be as described in any one of the embodiments of the present disclosure.

The present disclosure provides a preparation method of the compound of formula 4-2, comprising the following step S3: performing a reaction as shown below on the compound of formula 4-1 in a solvent in the presence of an organic base to obtain the compound of formula 4-2,

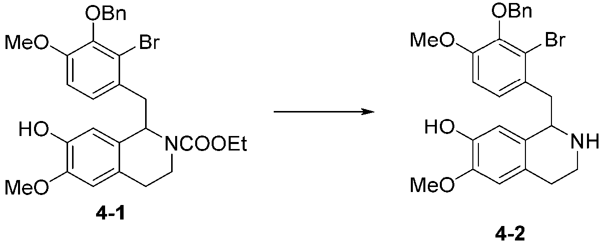

The organic base is potassium alkoxide and/or sodium alkoxide.

The conditions and operations for the preparation method of the compound of formula 4-2 may be as described in any one of the embodiments of the present disclosure.

The present disclosure provides a preparation method of the compound of formula 3, comprising the following step S1: performing a reaction as shown below on the compound of formula 1 and the compound of formula 2 in a solvent in the presence of an acid to obtain the compound of formula 3,

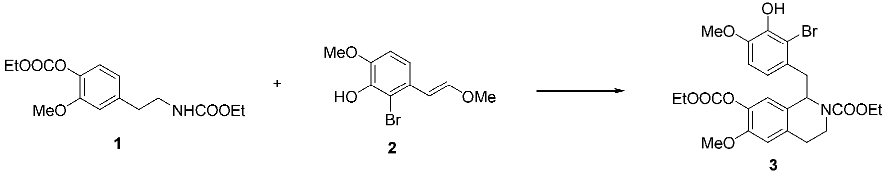

The acid is one or more of methanesulfonic acid, trifluoroacetic acid, p-toluenesulfonic acid, and p-toluenesulfonic acid monohydrate.

The conditions and operations for the preparation method of the compound of formula 3 may be as described in any one of the embodiments of the present disclosure.

The present disclosure provides a compound of formula 7, 6-2, 5, 4, 4-3, 4-1, 3, or 1.

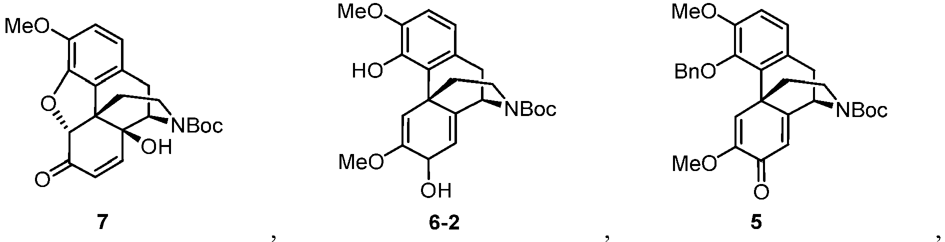

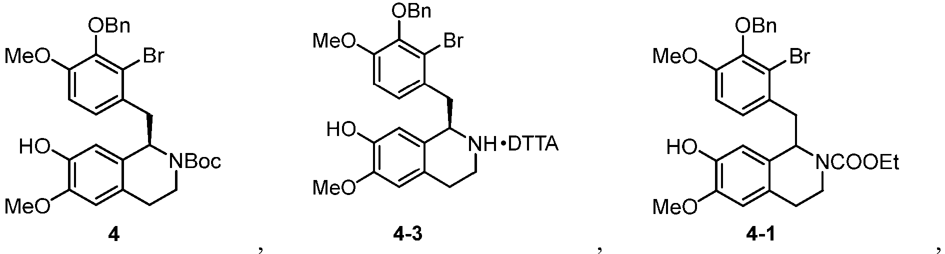

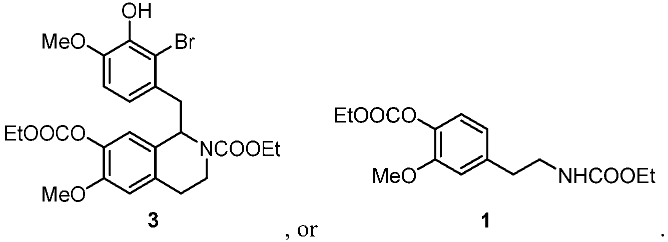

The present disclosure provides a use of the compound of formula 7 in the manufacture of naloxone, nalbuphine, oxycodone, or oxymorphone.

In the present disclosure, "room temperature" or "ambient temperature" refers to 10 to 30°C, for example, 25°C.

In the present disclosure, "ice-water bath" or "ice-salt bath" refers to -5 to 5°C, for example, 0°C.

Based on the common knowledge in the field, the above preferred conditions may be combined in any way to obtain the preferred embodiments of the present disclosure.

All reagents and raw materials used in the present disclosure are commercially available.

The positive and progressive effects of the present disclosure lie in that the intermediate provided by the present disclosure can be used to synthesize a variety of opioid derivatives and possesses one or more of the following advantages: low-cost and readily available raw materials, simplified synthesis steps, and high product yield.

### DETAILED DESCRIPTION OF THE PREFERRED EMBODIMENT

The present disclosure is further illustrated by way of the following examples, but it is not intended to limit the scope of the present disclosure to these examples. In the following examples, experimental methods without specified conditions are selected according to conventional methods and conditions or based on commercial instructions.

### Example 1: Synthesis of compound of formula 1

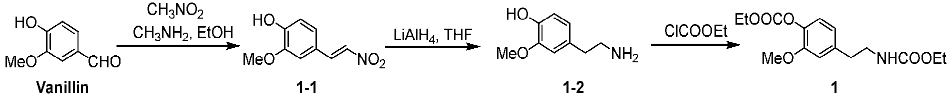

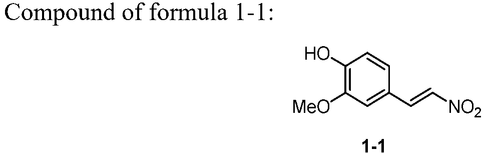

In a clean 100L reaction vessel, ethanol (75 L), vanillin (10.0 kg), methylamine hydrochloride (0.222 kg), and triethylamine (0.333 kg) were sequentially added at room temperature, resulting in a pale cyan solution with slight endothermicity. After stirring for 5 minutes, nitromethane (4.41 kg) was added, and the system immediately turned into an orange-red solution. It was stirred vigorously for 1 to 2 hours, followed by gentle stirring for 16 hours, during which a large amount of solid precipitated. HPLC indicated that vanillin was completely consumed, and the reaction was complete. The reaction mixture was filtered. The filter cake was washed with 2 L of ethanol once or twice, and then dried in an oven at 50°C for 24 hours to obtain the final product (compound I-1) as a dark yellow solid (10.3 kg, yield 82%).

¹H NMR (500 MHz, CDCl₃) δ 7.96 (d, J = 13.3 Hz, 1H), 7.52 (d, J = 13.5 Hz, 1H), 7.14 (d, J = 7.7 Hz, 1H), 7.04 - 6.94 (m, 2H), 6.05 (s, 1H), 3.96 (s, 3H).

¹³C NMR (126 MHz, CDCl₃) δ 149.88, 147.19, 139.63, 135.14, 125.07, 122.56, 115.42, 110.25, 56.24.

HRMS (ESI+): 194.0460.

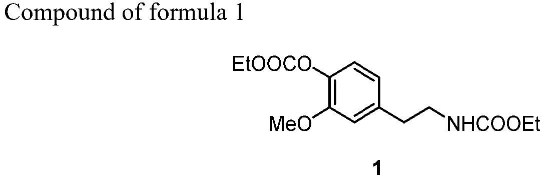

Under nitrogen atmosphere, 60 L of tetrahydrofuran was added to a 100 L reaction vessel, and 2 kg (10.25 mol) of compound 1-1 was dissolved in 30 L of tetrahydrofuran. Subsequently, 1.17 kg of lithium aluminum hydride in solid particulate form was added all at once to the reaction vessel at 5°C, and the mixture was stirred for 5 to 10 minutes until a white suspension formed. Subsequently, the tetrahydrofuran solution of compound 1-1 was pumped into the reaction vessel, with the temperature kept below 20°C. The system appeared as a yellow or golden suspension, which was then heated to 30°C and stirred for 16 hours. After the reaction was completed, the temperature in the reaction vessel was reduced to 0°C, and 3.51 L of water was slowly added dropwise to quench the reaction. The internal temperature was maintained below 20°C, and the system appeared as a pale yellow suspension. After the dropwise addition was completed, the system was stirred for 30 minutes, and then 2.46 kg (22.66 mol) of ethyl chloroformate was added at 0°C, with the system remaining a yellow suspension. After the dropwise addition was completed, nitrogen gas input was turned off, and the system was allowed to warm naturally for 10 minutes. The temperature in the reaction vessel was then raised to 25°C, and the mixture was stirred for 16 hours. After the reaction was completed, water was added thereto. The mixture was stirred for 1 to 2 hours, then filtered. The filtrate was washed once with 20 L of saturated brine and extracted with ethyl acetate (15 L). The organic phase was concentrated to obtain the product (compound 1) (2.9 kg, yield 90%).

¹H NMR (500 MHz, CDCl₃) δ 7.04 (d, J = 8.0 Hz, 1H), 6.82 - 6.71 (m, 2H), 4.73 (s, 1H), 4.30 (q, J = 7.1 Hz, 2H), 4.10 (dd, J = 13.8, 6.8 Hz, 2H), 3.83 (s, 3H), 3.42 (d, J = 6.2 Hz, 2H), 2.79 (t, J = 6.6 Hz, 2H), 1.37 (t, J = 7.1 Hz, 3H), 1.22 (t, J = 6.9 Hz, 3H).

¹³C NMR (126 MHz, CD₃OD) δ 156.7, 153.5, 151.2, 138.8, 138.1, 122.5, 120.9, 113.1, 65.0, 60.9, 56.0, 42.1, 36.2, 14.7, 14.3.

HRMS (ESI+):312.1447.

### Example 2: Compound of formula 2

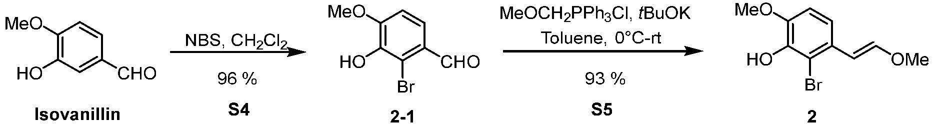

### Compound of formula 2-1

Isovanillin (10.0 g, 0.0657 mol, 1.0 eq.) was dissolved in 100 mL of dichloromethane (DCM) and cooled to 0°C. N-bromosuccinimide (NBS) (12.8 g, 0.0723 mol, 1.1 eq.) was added in batches and reacted at 0°C for 1 hour before returning to room temperature to continue the reaction. After the reaction was completed, the mixture was cooled to 10°C, and a saturated sodium chloride solution was added. The mixture was stirred for 0.5 hours and then filtered. The filter cake was washed twice with water and dried to obtain compound 2-1 (14.6 g, yield 96%). The organic phase in the filtrate from several batches can be combined and purified by recrystallization in petroleum ether/ethyl acetate (1:1) to obtain compound 2-1.

¹H NMR (500 MHz, CD₃OD) δ 10.20 (s, 1H), 7.47 (d, J = 8.6 Hz, 1H), 7.05 (d, J = 8.5 Hz, 1H), 3.96 (s, 3H).

¹³C NMR (126 MHz, CD₃OD) δ 191.20, 153.23, 127.05, 121.49, 113.22, 109.36, 55.52.

HRMS (ESI+): 230.9642.

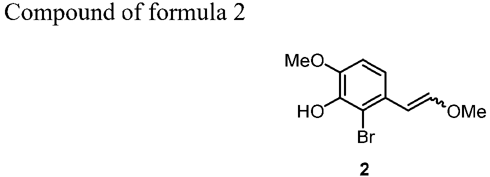

Under nitrogen atmosphere, toluene (50 L) was added into a 100 L reaction vessel with slow stirring, followed by the addition of potassium tert-butoxide (5 kg, 44.6 mol, 2.5 eq.). The system was cooled to 0°C, and (methoxymethyl)triphenylphosphonium chloride (13.75 kg, 40.18 mol, 2.25 eq.) was added while maintaining the internal temperature below 5°C. The mixture was then heated to 25°C and reacted for 3 hours. The system was cooled to 0°C, and compound 2-1 (4.11 kg, 17.86 mol, 1.0 eq.) was added slowly. The system was then allowed to warm slowly to room temperature and reacted for 16 hours. After the reaction was completed, the mixture was cooled to 0°C and quenched by slowly adding water (20 L) while adding KOH (500 g) at the same time. The mixture was separated, and the toluene phase was removed. The aqueous phase was adjusted to weakly acidic pH with ammonium chloride, then extracted twice with ethyl acetate, concentrated to obtain the crude product 2 as a golden-yellow oil (4.28 kg, yield 93%).

¹H NMR (500 MHz, CDCl₃) δ 7.60 (d, J = 8.7 Hz, 1H), 6.90 - 6.84 (m, 2H), 6.77 (dd, J = 19.2, 8.6 Hz, 2H), 6.19 (d, J = 7.2 Hz, 1H), 6.02 (d, J = 12.8 Hz, 1H), 5.95 (d, J = 16.6 Hz, 2H), 5.54 (d, J = 7.2 Hz, 1H), 3.89 (d, J = 3.3 Hz, 6H), 3.76 (s, 3H), 3.71 (s, 3H).

### Example 3: Compound of formula 3

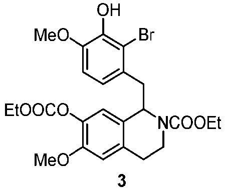

Into a clean and dry round-bottom flask equipped with a stir bar were added compound 1 (36.4 g, 0.117 mol, 1.0 equiv), compound 2 (42.3 g, 0.164 mol, 1.4 equiv), and dichloromethane (150 mL), followed by the addition of p-toluenesulfonic acid monohydrate (45.7 g, 0.24 mol, 2.0 equiv) at room temperature. The mixture was stirred for 16 hours. After the reaction was completed, the mixture was quenched with water. The organic phase was dried over anhydrous sodium sulfate and concentrated to obtain the crude product. The crude product was recrystallized from a mixed solvent of ethyl acetate/petroleum ether (2:1) to obtain product 3 (50.2 g, yield 80%).

¹H NMR (500 MHz, CDCl₃) δ 7.08 (s, 1H), 6.77 (s, 1H), 6.71 (d, J = 5.4 Hz, 2H), 6.68 (d, J = 3.0 Hz, 1H), 6.61 (d, J = 8.3 Hz, 1H), 6.56 (d, J = 8.2 Hz, 1H), 6.02 (s, 1H), 5.96 - 5.89 (m, 1H), 5.45 - 5.39 (m, 1H), 4.40 - 4.26 (m, 4H), 4.07 - 4.02 (m, 1H), 4.00 - 3.95 (m, 1H), 3.87 (s, 3H), 3.86 (s, 1H), 3.83 (s, 3H), 3.82 (s, 1H), 3.75 (dt, J = 10.4, 7.2 Hz, 1H), 3.57 - 3.48 (m, 1H), 3.41 (s, 1H), 3.33 - 3.20 (m, 2H), 3.19 - 3.09 (m, 1H), 2.93 (dt, J = 11.7, 8.8 Hz, 2H), 2.85 - 2.80 (m, 1H), 2.72 (d, J = 15.1 Hz, 1H), 2.68 (s, 1H), 1.43 - 1.34 (m, 4H), 1.19 (d, J = 7.1 Hz, 1H), 0.92 (t, J = 7.1 Hz, 3H).

¹³C NMR (126 MHz, CDCl₃) δ 155.6, 153.5, 149.9, 145.9, 143.1, 138.5, 133.1, 130.6, 129.1, 122.0, 120.8, 112.7, 111.5, 109.4, 65.1, 61.3, 56.5, 56.1, 53.2, 42.2, 36.6, 28.8, 14.3, 14.1.
HRMS (ESI+): 560.0895

### Example 4: Compound of formula 4

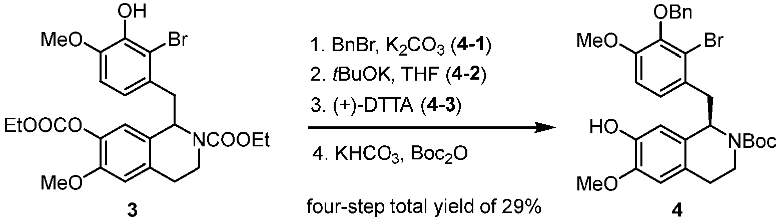

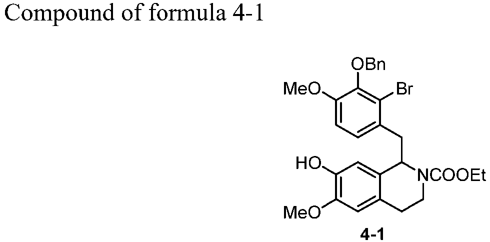

Under nitrogen atmosphere, N,N-dimethylformamide (DMF) (20 L)was added to a clean and dry 100 L reaction vessel with slow stirring. Compound 3 (7.8 kg, 12.4 mol, 1.0 equiv) was added, and potassium carbonate (2.78 kg, 24.8 mol, 2.0 equiv) and benzyl bromide (1.39 kg, 24.8 mol, 2.0 equiv) were sequentially added thereto at room temperature. The reaction mixture was reacted for 6 hours at room temperature. After the reaction was completed, the system was added with water (40 L) to precipitate the solid product and filtered to obtain a brown solid. The brown solid was directly dissolved in ethanol (20 L), then 20 g of solid KOH (2.0 equiv) was added. The system was heated to 50°C and stirred for 3 hours. After the reaction was completed, the mixture was cooled to room temperature, added with water (40 L), and stirred for 2 hours. The system was filtered, and the filter cake was slurried with methanol. The mixture was filtered again, and the filter cake was dried to obtain the product 4-1 (8.0 kg) as a gray solid.

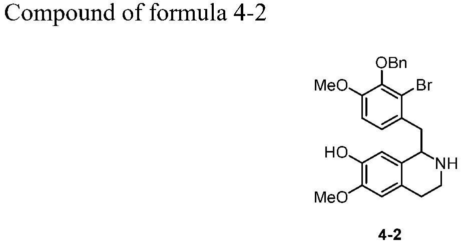

Under nitrogen atmosphere, tetrahydrofuran (40 L) was added to a clean and dry 100 L reaction vessel with slow stirring. Compound 4-1 (7.8 kg, 12.4 mol, 1.0 equiv) was added, and potassium tert-butoxide (2.78 kg, 24.8 mol, 2.0 equiv) and potassium hydroxide (1.39 kg, 24.8 mol, 2.0 equiv) were added at room temperature. The reaction mixture was heated to 70°C and reacted for 8 hours. After the reaction was completed, the system was cooled to 10°C, and the reaction was quenched with water (15 L). The system was extracted three times with dichloromethane (15 L × 3). The solvent was evaporated to obtain a crude product 4-2 (5.85 kg) as a pale yellow foamy solid.

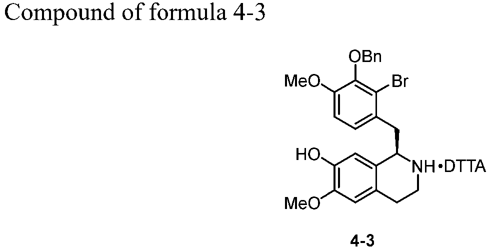

Into a clean and dry 100L reaction vessel were added crude compound 4-2 (5.85 kg, 12.4 mol, 1.0 equiv) and methanol (20 L). D-(+)-di-p-toluoyl tartaric acid ((+)-DTTA) (5.0 kg, 13.0 mol, 1.05 equiv) was added at room temperature. The reaction mixture was heated to 85°C and reacted for 8 hours. After the reaction was completed, the mixture was directly filtered to obtain the product 4-3 (3.2 kg, 95% ee) as a white solid (Chiral HPLC conditions: OD-H, i-PrOH/hexane = 80:20, flow rate 1.0 mL/min, 210 nm. t1 = 6.5 min, t2 = 8.0 min, T = 15.00 min.)

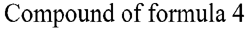

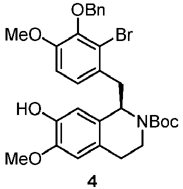

Into a clean 50 L reaction vessel were added dichloromethane (30 L) and compound 4-3 (3.2 kg, 3.79 mol, 1.0 equiv.), followed by the addition of an aqueous solution of potassium bicarbonate KHCO₃ (1.9 kg, 19 mol, 5.0 equiv.) under stirring. The system was stirred at room temperature for 1 hour, and the dichloromethane phase was collected. The aqueous phase was extracted twice with dichloromethane (5 L × 2). The dichloromethane phases were combined and transferred to another clean reaction vessel. The system was cooled to 20°C, and di-tert-butyl dicarbonate (Boc₂O) (871 g, 4.0 mol, 1.08 equiv) was added. The reaction was maintained at room temperature for 3 hours. After the reaction was completed, most of the dichloromethane was evaporated and replaced with methanol, precipitating a solid. The solid was filtered and dried to obtain the product 4 (2.07 kg, yield 96%) as a white sand-like substance.

¹H NMR (400 MHz, CDCl₃) δ 7.59 (d, J = 7.2 Hz, 2H), 7.40 (t, J = 7.4 Hz, 2H), 7.34 (t, J = 7.3 Hz, 1H), 6.97 (s, 1H), 6.81 (s, 2H), 6.61 (s, 1H), 5.59 (s, 1H), 5.32 (dd, J = 11.0, 2.9 Hz, 1H), 5.05 (s, 2H), 4.38 (dd, J = 13.3, 5.2 Hz, 1H), 3.88 (s, 3H), 3.84 (s, 3H), 3.29 - 3.23 (m, 2H), 2.97 - 2.88 (m, 2H), 2.64 (dd, J = 16.1, 2.0 Hz, 1H), 1.11 (s, 9H).

¹³C NMR (101 MHz, CDCl₃) δ 154.6, 152.7, 145.6, 145.4, 144.1, 137.4, 131.3, 130.1, 128.5, 128.4, 128.1, 126.5, 125.9, 121.4, 112.8, 111.3, 110.9, 79.5, 74.7, 56.3, 56.1, 53.7, 42.3, 36.2, 28.5, 28.5, 28.1, 27.8.

HRMS (ESI+):584.1652.

### Example 5: Compound of formula 5

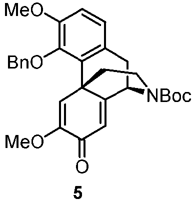

Into a clean and dry 250 mL Schlenk tube were added palladium chloride (60 mg, 0.34 mmol), bis(1-adamantyl)butylphosphine (168 mg, 0.52 mmol), potassium carbonate (2.37 g, 17.2 mmol), and compound 4 (5 g, 8.6 mmol). The entire system was purged with nitrogen three times, followed by the addition of N,N-dimethylacetamide (30 mL). The entire reaction system was stirred at 140°C for 6 hours. After the reaction was completed, the system was cooled to room temperature, and dichloromethane (100 mL) was added. The crude mixture was filtered through diatomite, concentrated, and purified by column chromatography (eluent: dichloromethane/methanol = 150:1 to 100:1) to obtain the product compound 5 (2.3 g, yield 53%) as a yellow solid.

¹H NMR (400 MHz, CDCl₃) δ 7.50 (d, J = 6.9 Hz, 2H), 7.41 (t, J = 7.3 Hz, 2H), 7.35 (t, J = 7.0 Hz, 1H), 6.88 (s, 2H), 6.31 (d, J = 20.1 Hz, 1H), 5.33 (d, J = 11.1 Hz, 1H), 5.15 - 4.89 (m, 2H), 3.96 - 3.77 (m, 4H), 3.40 (s, 3H), 3.16 (tt, J = 29.0, 14.5 Hz, 2H), 2.87 - 2.70 (m, 1H), 2.34 (d, J = 12.4 Hz, 1H), 1.47 (d, J = 29.0 Hz, 9H).

¹³C NMR (101 MHz, CDCl₃) δ 181.1, 159.8, 154.7, 152.0, 151.6, 146.4, 137.5, 130.9, 128.8, 128.2, 127.4, 127.3, 124.5, 122.0, 121.8, 120.3, 120.1, 112.3, 80.6, 80.5, 74.3, 56.1, 54.9, 54.0, 52.8, 44.1, 39.5, 39.0, 38.9, 38.8, 29.8, 28.5.

HRMS (ESI+): 504.2388.

### Example 6: Compound of formula 6

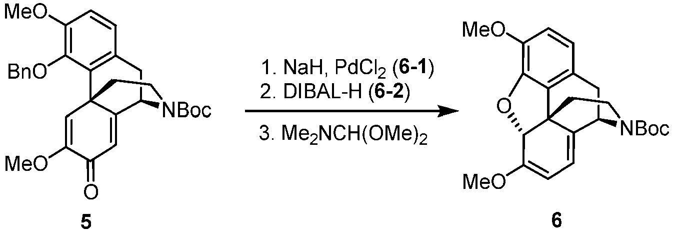

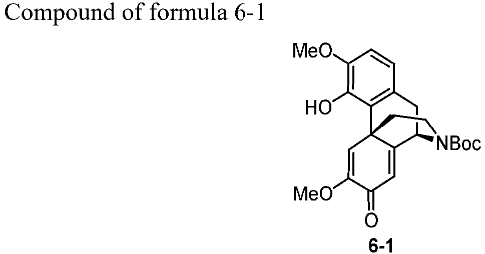

Under nitrogen atmosphere, palladium chloride (1.76 g, 10 mmol) and sodium hydride (12 g, 0.3 mol, 60% in mineral oil) were added to a clean and dry 1 L three-neck round-bottom flask and stirred for 30 minutes. The system was cooled to 0°C, followed by the addition of a solution of 5 (100 g, 0.2 mol) in N,N-dimethylacetamide (DMAc) (500 mL). The entire reaction system can be heated to 25°C and stirred for 10 hours. After the reaction was completed, the system was cooled to 0°C, and water (200 mL) was added to quench the reaction. The crude mixture was extracted three times with ethyl acetate (300 mL × 3). The organic phases were combined, dried over anhydrous sodium sulfate, and concentrated to obtain the crude product 6-1 (80 g) as a brown oil.

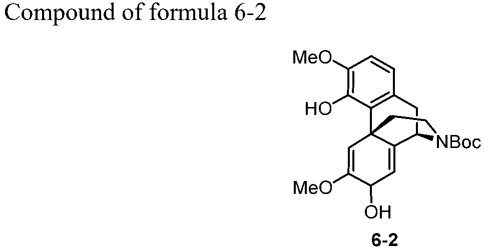

Under nitrogen atmosphere, the crude compound 6-1 (80 g, 0.19 mol, 1.0 equiv) and tetrahydrofuran (600 mL) were added to a clean and dry 1 L three-neck round-bottom flask. The system was cooled to -20°C, and a tetrahydrofuran (THF) solution of diisobutylaluminum hydride (DIBAL-H) (258 mL, 0.39 mol, 2.0 equiv) was slowly added while maintaining the internal temperature below 5°C. After the addition was completed, the reaction system was warmed to 15°C and reacted for 30 minutes. After the reaction was completed, the system was poured into a cooled aqueous NaOH solution (4 N) to quench the reaction. The mixture was extracted five times with dichloromethane (500 mL × 5). The organic phases were combined, dried over anhydrous sodium sulfate, and concentrated to obtain the crude product 6-2 (75.4 g) as a brown oil.

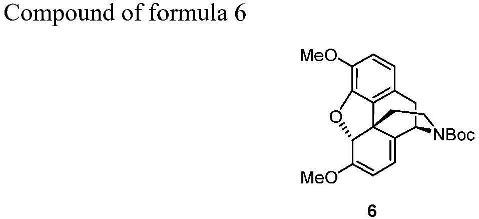

Under nitrogen atmosphere, compound 6-2 (75.4 g, 0.18 mol, 1.0 equiv) was added to a clean and dry round-bottom flask, followed by the addition of dichloromethane (200 mL). At room temperature, N,N-dimethylformamide dimethyl acetal (DMFDMA) (35.6 mL, 0.27 mol, 1.5 equiv) was added, and the system was allowed to react at room temperature for 3 hours. After the reaction was completed, the mixture was directly concentrated, extracted three times with a mixed solvent of petroleum ether/ethyl acetate (6:1). The organic phases were combined and evaporated to dryness, and then the product 6 (27.6 g, yield 35%) was precipitated under methanol/water (4:1) at -10°C.

¹H NMR (500 MHz, CDCl₃) δ 6.68 - 6.63 (m, 1H), 6.57 (d, J = 8.1 Hz, 1H), 5.57 (d, J = 24.6 Hz, 1H), 5.27 (d, J = 4.3 Hz, 1H), 5.18 - 4.95 (m, 2H), 4.01 (dd, J = 60.2, 13.4 Hz, 1H), 3.87 - 3.80 (m, 3H), 3.62 - 3.54 (m, 3H), 3.27 - 3.04 (m, 2H), 2.97 (d, J = 18.0 Hz, 1H), 2.03 (d, J = 12.0 Hz, 1H), 1.76 (s, 1H), 1.47 (d, J = 11.0 Hz, 9H).

¹³C NMR (126 MHz, CDCl₃) δ 154.4, 152.9 (d, J = 26.2 Hz), 144.9, 143.1, 132.6, 130.5 (d, J = 29.1 Hz), 126.7 (d, J = 23.7 Hz), 119.6, 113.2 (d, J = 10.9 Hz).111.8, 111.5, 95.9, 95.7, 88.9, 79.9, 56.4, 55.1, 53.2, 51.7, 46.4, 38.2, 37.9, 37.3, 36.9, 28.5.

HRMS (ESI+): 398.1970.

### Example 7: Compound of formula 7

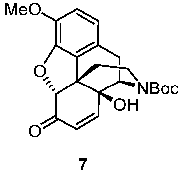

Into a clean and dry round-bottom flask was added compound 6 (10 g, 25.2 mmol, 1.0 equiv), followed by the addition of a mixed solvent of dichloromethane/acetic acid/water (7:2:1) (200mL). The system was cooled to 0°C, and magnesium monoperoxyphthalate hexahydrate (MMPP) (14.6 g, 37.8 mmol, 1.5 equiv) was added. The system was then warmed to 15°C and reacted for 3 hours. After the reaction was completed, an aqueous solution of sodium thiosulfate was added to quench the reaction, and the system was extracted three times with dichloromethane (100 mL × 3). The organic phases were combined, dried over anhydrous sodium sulfate, concentrated, and purified by column chromatography (eluent: dichloromethane/ethyl acetate = 4:1) to afford the product 7 as a yellow foamy solid (8.94 g, yield 89%).

Optical rotation [α]_{D}²⁵ = -164.3° (c 1.0 CHCl₃).

¹H NMR (500 MHz, CDCl₃) δ 6.75 - 6.69 (m, 2H), 6.63 (d, J = 8.2 Hz, 1H), 6.16 (d, J = 10.1 Hz, 1H), 4.72 (s, 2H), 3.97 (s, 1H), 3.85 (s, 3H), 2.98 (d, J = 3.4 Hz, 2H), 2.84 (s, 1H), 2.46 (s, 1H), 1.50 (s, 9H).

¹³C NMR (126 MHz, CDCl₃) δ 194.4, 144.4, 143.0, 133.7, 130.1, 124.3, 120.1, 119.7, 115.3, 111.6, 109.2, 86.8, 80.9, 68.1, 63.6, 56.8, 56.3, 54.4, 47.3, 31.6, 29.7, 28.5, 27.6.

HRMS (ESI+): 400.1766.

Example 8: Compound of formula 8

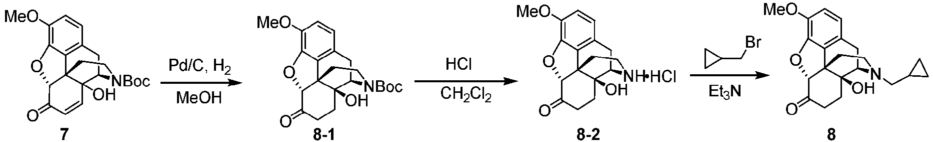

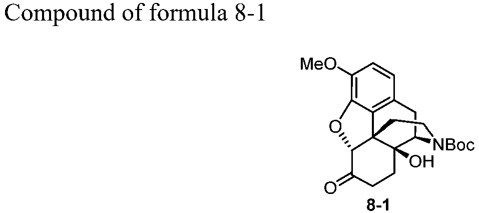

Into a clean and dry 50 mL round-bottom flask were added compound 7 (500 mg, 1.25 mmol) and palladium on carbon (50 mg, 10% Pd on carbon), followed by the addition of methanol (10 mL). The entire system was placed in a high-pressure reaction vessel, purged with hydrogen three times until the hydrogen pressure reached 2 atm, and reacted at 30°C for 4 hours under this pressure condition. After the reaction was completed, the mixture was filtered through diatomite to recover the palladium on carbon, and the filtrate was concentrated directly to obtain the crude product 8-1 (500 mg) as a pale green foam.

¹H NMR (500 MHz, CDCl₃) δ 6.74 (d, J = 8.2 Hz, 1H), 6.65 (d, J = 8.0 Hz, 1H), 4.67 (s, 1H), 4.52 (s, 1H), 3.90 (d, J = 2.2 Hz, 3H), 3.11 - 2.96 (m, 2H), 2.90 (d, J = 18.2 Hz, 1H), 2.72 (s, 2H), 2.41 (s, 1H), 2.30 (d, J = 14.3 Hz, 1H), 1.92 (d, J = 12.1 Hz, 1H), 1.66 (d, J = 13.1 Hz, 2H), 1.50 (s, 9H).

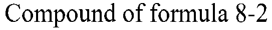

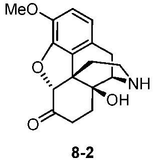

In a clean three-neck flask were added the crude compound 8-1 (450 mg, 1.12 mmol) and methanol (10 mL). The system was cooled to 0°C, then a solution of HCl in methanol (4 N, 12 mL) was slowly added. The internal temperature of the system was maintained below 10°C. After the reaction was completed (about 1.5 hours), the system was concentrated directly at 30°C to obtain the crude product 8-2 (410 mg) as a pale yellow solid.

¹H NMR (500 MHz, DMSO-*d*₆) δ 9.66 (d, J = 10.8 Hz, 1H), 8.54 (s, 1H), 6.84 (d, J = 8.2 Hz, 1H), 6.76 (d, J = 8.2 Hz, 1H), 6.54 (s, 1H), 5.00 (s, 1H), 3.80 (s, 3H), 3.70 (s, 1H), 3.31 (d, J = 19.2 Hz, 1H), 3.20 (d, J = 29.8 Hz, 1H), 3.14 - 2.89 (m, 3H), 2.10 (d, J = 14.4 Hz, 1H), 1.95 (d, J = 13.2 Hz, 1H), 1.43 (dd, J = 21.5, 11.7 Hz, 2H).

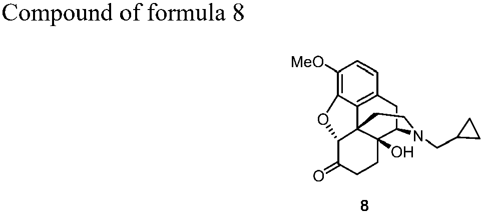

Into a clean and dry Schlenk tube was added the crude compound 8-2 (380 mg, 1.12 mmol). After purging with nitrogen three times, 6 mL of N-methylpyrrolidone and water (NMP:H₂O=5:1) were added. Triethylamine (0.78 mL, 5.6 mmol) was added at room temperature, and the mixture was stirred for 5 minutes. Subsequently, cyclopropylmethyl bromide (0.14 mL, 1.5 mmol) was added. The entire system was reacted at 70°C for 4 hours. After completion of the reaction, the mixture was cooled to room temperature, and water (15 mL) was added. The system was extracted three times with dichloromethane (20 mL × 3). The organic phase was dried, concentrated, and purified by column chromatography (eluent: dichloromethane/methanol=50:1) to afford compound 8 (286 mg, yield 72%) as a white solid.

¹H NMR (500 MHz, CDCl₃) δ 6.68 (d, J = 8.1 Hz, 1H), 6.60 (d, J = 8.1 Hz, 1H), 4.68 (s, 1H), 3.87 (s, 3H), 3.29 (s, 1H), 3.03 (t, J = 14.5 Hz, 2H), 2.75 (s, 2H), 2.61 (d, J = 15.6 Hz, 1H), 2.46 (s, 3H), 2.28 (d, J = 14.3 Hz, 1H), 2.21 - 2.09 (m, 1H), 1.91 (s, 1H), 1.67 - 1.51 (m, 2H), 0.90 (s, 1H), 0.56 (d, J = 6.8 Hz, 2H), 0.17 (s, 2H).

¹³C NMR (101 MHz, CDCl₃) δ 208.53, 145.15, 143.07, 129.63, 124.94, 119.49, 115.18, 90.50, 70.27, 62.21, 59.32, 57.00, 50.88, 43.81, 36.26, 31.62, 30.74, 29.78, 22.80, 9.43, 4.11, 3.94.

HRMS (ESI+): 356.1860.

### Example 9: Compound of formula 9 (Naltrexone)

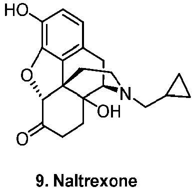

Into a clean and dry Schlenk tube was added compound 8 (80 mg, 0.225 mmol). The system was purged with nitrogen three times, and dry dichloromethane (5 mL) was added. The entire system was cooled to -10°C, followed by the slow dropwise addition of boron tribromide (0.15 mL, 1.6 mmol). The system was then warmed to 10°C and stirred for 2 hours. After the reaction was completed, the mixture was cooled to 0°C and saturated ammonium chloride (5 mL) was added. Subsequently, saturated 10% NaHCO₃ was added to adjust the pH to 8. The system was extracted four times with dichloromethane (20 mL × 4). The organic phases were combined, dried over anhydrous sodium sulfate, concentrated, and purified by column chromatography (eluent: a mixed solvent of dichloromethane/methanol at 50:1) to obtain naltrexone (58 mg, yield 76%) as a white solid.

¹H NMR (500 MHz, CDCl₃) δ 6.72 (d, J = 8.0 Hz, 1H), 6.58 (d, J = 7.9 Hz, 1H), 4.69 (s, 1H), 3.20 (d, J = 4.8 Hz, 1H), 3.11 - 2.98 (m, 2H), 2.71 (d, J = 7.7 Hz, 1H), 2.57 (dd, J = 18.3, 5.5 Hz, 1H), 2.49 - 2.37 (m, 3H), 2.32 (d, J = 14.4 Hz, 1H), 2.17 (dd, J = 12.1, 9.1 Hz, 1H), 1.89 (d, J = 13.1 Hz, 1H), 1.62 (ddd, J = 27.4, 19.5, 6.8 Hz, 2H), 0.87 (d, J = 6.9 Hz, 1H), 0.55 (d, J = 7.6 Hz, 2H), 0.15 (d, J = 4.5 Hz, 2H).

¹³C NMR (126 MHz, CDCl₃) δ 210.13, 143.60, 138.95, 129.11, 124.34, 120.07, 118.09, 90.72, 70.38, 62.17, 59.35, 51.18, 43.76, 36.33, 31.51, 30.77, 29.84, 22.78, 9.52, 4.15, 3.96.

HRMS (ESI+): m/z calculated for C₂₀H₂₄NO₄ [M+H]⁺: 342.1700, found: 342.1703.

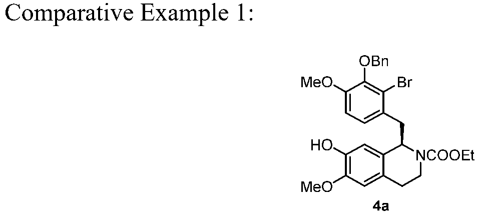

In the same manner as for the synthesis of compound 4, an aqueous solution of potassium bicarbonate KHCO₃ was added to a dichloromethane solution of compound 4-3. The mixture was stirred for 1 hour, then the dichloromethane phase was separated. Ethyl chloroformate ClCOOEt (1.05 equiv) was added, and upon completion of the reaction, water was added to quench the reaction, followed by phase separation. The organic phase was dried and concentrated under reduced pressure, and purified by column chromatography (eluent: petroleum ether/ethyl acetate = 1:1) to obtain compound 4a.

¹H NMR (500 MHz, CDCl₃) δ 7.58 (dd, J = 16.2, 7.3 Hz, 2H), 7.37 (ddd, J = 29.4, 13.9, 6.9 Hz, 3H), 6.92 (s, 1H), 6.78 (d, J = 8.1 Hz, 2H), 6.63 - 6.55 (m, 1H), 5.50 (d, J = 46.1 Hz, 1H), 5.41 - 5.34 (m, 1H), 5.00 (d, J = 23.7 Hz, 2H), 4.35 (dd, J = 13.0, 4.9 Hz, 1H), 4.05 (dd, J = 7.1, 3.4 Hz, 1H), 3.94 - 3.80 (m, 6H), 3.80 - 3.74 (m, 1H), 3.56 - 3.38 (m, 1H), 3.34 -3.09 (m, 2H), 3.00 - 2.74 (m, 2H), 2.64 (t, J = 17.3 Hz, 1H), 1.06 (dt, J = 125.0, 7.1 Hz, 3H).

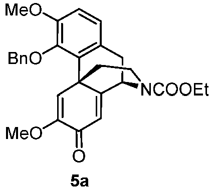

Following the same procedure as for compound 5, into a clean and dry 250 mL three-neck flask were added palladium chloride (60 mg, 0.34 mmol), bis(1-adamantyl)butylphosphine (168 mg, 0.52 mmol), potassium carbonate (2.37 g, 17.2 mmol), and compound 4a (4.75 g, 8.6 mmol). The entire system was purged with nitrogen three times, followed by the addition of N,N-dimethylacetamide (30 mL). The entire reaction system was stirred at 140°C for 6 hours. After the reaction was completed, the system was cooled to room temperature, and dichloromethane (100 mL) was added. The crude mixture was filtered through diatomite, concentrated, and purified by column chromatography (eluent: dichloromethane/methanol = 150:1 to 100:1) to obtain the product compound 5a (2.2 g, yield 55%) as a yellow solid.

¹H NMR (500 MHz, CDCl₃) δ 7.41 (d, J = 8.6 Hz, 2H), 7.30 (s, 1H), 6.93 (d, J = 8.6 Hz, 2H), 6.87 (s, 2H), 6.30 (d, J = 15.8 Hz, 1H), 5.29 (s, 2H), 5.14 (s, 1H), 5.01 (s, 1H), 4.96 (d, J = 10.8 Hz, 1H), 4.24 - 4.09 (m, 2H), 3.89 (s, 3H), 3.83 (s, 3H), 3.46 (s, 3H), 3.25 - 3.06 (m, 2H), 2.91 (d, J = 36.1 Hz, 1H), 2.82 (s, 1H), 2.34 (dd, J = 13.0, 2.3 Hz, 1H), 1.59 - 1.50 (m, 1H), 1.32 (t, J = 6.9 Hz, 1H), 1.27 - 1.21 (m, 2H).

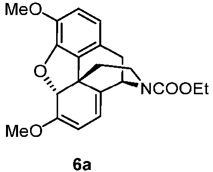

Similarly to the synthesis of compound 6, compound 6a was obtained from compound 5a under the same conditions.

¹H NMR (500 MHz, CDCl₃) δ 6.68 (d, J = 8.2 Hz, 1H), 6.60 (d, J = 8.1 Hz, 1H), 5.61 (dd, J = 20.7, 6.1 Hz, 1H), 5.14 (dd, J = 71.3, 6.4 Hz, 1H), 5.04 (d, J = 6.4 Hz, 1H), 4.24 - 4.00 (m, 4H), 3.85 (s, 3H), 3.70 (s, 1H), 3.60 (s, 3H), 3.32 - 3.08 (m, 2H), 3.05 - 2.98 (m, 1H), 2.08 (dt, J = 12.5, 6.1 Hz, 1H), 1.81 (t, J = 12.1 Hz, 1H), 1.27 (dd, J = 16.1, 8.9 Hz, 4H).

During the synthesis of naltrexone from compound 6a, the inventors attempted numerous conditions but never succeeded due to the failure of removing the -COOEt protecting group. When the conditions for removing the -COOEt protecting group in the preparation method of the compound of formula 4-2 in Example 4 were applied, the skeleton of compound 6a was unstable, resulting in numerous by-products and a very low yield of the target compound.

The inventors also attempted to remove the protecting group under acidic and other basic conditions, which similarly resulted in skeleton instability, yielding almost no target product or leading to extremely low yields.

From the above comparative examples, it can be seen that using tert-butoxycarbonyl (Boc) as a protecting group not only facilitates easy removal under acidic conditions with stable skeleton, but also enhances the yield of the target product and simplifies subsequent functional group transformations.

## Claims

1. A preparation method of a compound of formula A8, comprising the following steps S11, S12, and S13:
S11: performing a hydrogenation reaction as shown below on a compound of formula 7 with hydrogen in a solvent in the presence of a catalyst to obtain a compound of formula 8-1; 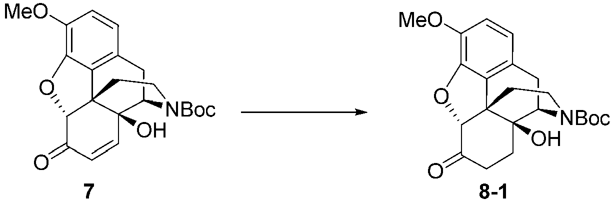
S12: performing a deprotection reaction as shown below on the compound of formula 8-1 in a solvent in the presence of a deprotecting reagent to obtain a compound of formula 8-2; 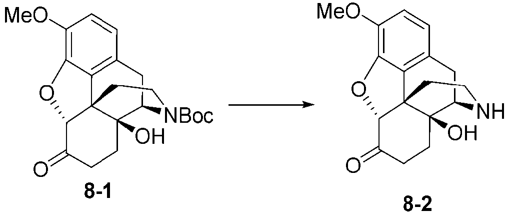
S13: performing a substitution reaction as shown below on the compound of formula 8-2 with a compound of formula SM1 in a solvent in the presence of a base to obtain a compound of formula A8; 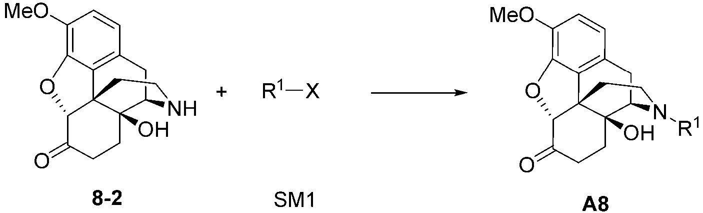
wherein X is halogen, and R¹ is C₁₋₄ alkyl or C₁₋₄ alkyl substituted with one or more R¹⁻¹; R¹⁻¹ is 3- to 6-membered cycloalkyl or C₂₋₄ alkenyl.

2. The preparation method of the compound of formula A8 according to claim 1, wherein the preparation method satisfies one or more of the following conditions:
(1) in X, the halogen is F, Cl, Br, or I, for example, Br;
(2) in said R¹, the C₁₋₄ alkyl in said C₁₋₄ alkyl and said C₁₋₄ alkyl substituted with one or more R¹⁻¹ is independently methyl, ethyl, n-propyl, isopropyl, tert-butyl, n-butyl, or isobutyl, for example, methyl;
(3) in said R¹, the C₁₋₄ alkyl substituted with one or more R¹⁻¹ is C₁₋₄ alkyl substituted with one R¹⁻¹;
(4) in said R¹⁻¹, the 3- to 6-membered cycloalkyl is cyclopropyl, cyclobutyl, cyclopentyl, or cyclohexyl, for example, cyclopropyl or cyclobutyl; and
(5) in said R¹⁻¹, the C₂₋₄ alkenyl is vinyl, propenyl, or butenyl, for example, vinyl.

3. The preparation method of the compound of formula A8 according to claim 2, wherein the preparation method satisfies one or two of the following conditions:
(1) X is Br; and
(2) R¹ is methyl, 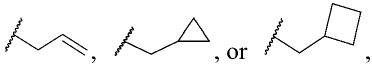 for example,

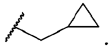

4. The preparation method of the compound of formula A8 according to any one of claims 1 to 3, wherein the preparation method satisfies one or more of the following conditions:
(1) in step S11, the solvent is an alcohol solvent, for example, methanol and/or ethanol, for another example, methanol;
(2) in step S11, the molar volume ratio of the compound of formula 7 to the solvent is (0.1 to 0.3) mol: 1 L, for example, 0.13 mol: 1 L;
(3) in step S11, the catalyst is Raney nickel and/or a palladium catalyst, for example, a palladium catalyst, for another example, 10% palladium on carbon, where "%" represents the mass percentage of palladium relative to the total mass of palladium and carbon;
(4) in step S11, the mass ratio of the catalyst to the compound of formula 7 is (0.01 to 1):1, for example, (0.05 to 0.5):1, for another example, 0.1:1;
(5) in step S11, the pressure of the hydrogenation reaction is 1 to 10 atm, for example, 1 to 3 atm, for another example, 2 atm;
(6) in step S11, the temperature of the hydrogenation reaction is 10 to 45°C, for example, 20 to 30°C, for another example, 30°C;
(7) in step S11, the duration of the hydrogenation reaction is 1 to 10 hours, for example, 3 to 6 hours, for another example, 4 hours;
(8) in step S11, after completion of the hydrogenation reaction, the following work-up steps are comprised: filtration and concentration;
(9) in step S11, the next reaction step is performed without a purification step following completion of the hydrogenation reaction;
(10) in step S12, the solvent is an alcohol solvent, for example, methanol and/or ethanol, for another example, methanol;
(11) in step S12, the molar volume ratio of the compound of formula 8-1 to the solvent is (0.01 to 1) mol: 1 L, for example, 0.05 mol: 1 L;
(12) in step S12, the deprotecting reagent is an acid, for example, an inorganic acid, for another example, HCl, for yet another example, a 4 mol/L solution of HCl in methanol;
(13) in step S12, the molar ratio of the acid to the compound of formula 8-1 is (1 to 100):1, for example, (30 to 60):1, for another example, 43:1;
(14) in step S12, the temperature of the deprotection reaction is 0 to 20°C, for example, 10°C;
(15) in step S12, the duration of the deprotection reaction is 0.5 to 3 hours, for example, 1.5 hours;
(16) in step S12, after completion of the deprotection reaction, the following work-up step is comprised: concentration;
(17) in step S12, the next reaction step is performed without a purification step following completion of the deprotection reaction;
(18) the compound of formula SM1 is 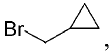 and the compound of formula A8 is 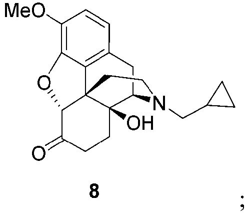
(19) in step S13, the solvent is a mixed solvent of an organic solvent and water; the organic solvent may be a pyrrolidone organic solvent, for example, N-methylpyrrolidone; the volume ratio of the organic solvent to water may be (1 to 10):1, for example, (4 to 6):1, for another example, 5:1;
(20) in step S13, the molar volume ratio of the compound of formula 8-2 to the solvent is (0.01 to 0.3) mol: 1 L, for example, 0.19 mol: 1 L;
(21) in step S13, the base is an organic base, for example, an organic amine, for another example, triethylamine;
(22) in step S13, the molar ratio of the base to the compound of formula 8-2 is (1 to 10):1, for example, (4 to 6):1, for another example, 5:1;
(23) in step S13, the molar ratio of the compound of formula SM1 to the compound of formula 8-2 is (1 to 3):1, for example, (1 to 1.5): 1, for another example, 1.3:1;
(24) in step S13, the substitution reaction is performed under the atmosphere of an inert gas, which may be nitrogen and/or argon, for example, nitrogen;
(25) in step S13, the temperature of the substitution reaction is 60 to 90°C, for example, 70°C;
(26) in step S13, the duration of the substitution reaction is 1 to 6 hours, for example, 4 hours; and
(27) in step S13, after completion of the substitution reaction, one or more of the following work-up steps are comprised: extraction, drying, concentration, and purification; the solvent for said extraction may be dichloromethane; the desiccant for said drying may be anhydrous sodium sulfate; said concentration may be distillation under reduced pressure; said purification may be column chromatography; the eluent for said column chromatography may be a mixed solvent of dichloromethane and methanol, for example, a mixed solvent with a volume ratio of dichloromethane to methanol of 50:1.

5. The preparation method of the compound of formula A8 according to claim 1, wherein the preparation method of the compound of formula A8 further comprises a preparation method of the compound of formula 7, specifically comprising the following step S10: performing an oxidation reaction as shown below on a compound of formula 6 in a solvent in the presence of an oxidant to obtain the compound of formula 7,

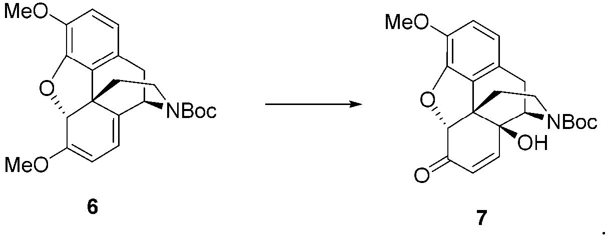

6. The preparation method of the compound of formula A8 according to claim 5, wherein the preparation method satisfies one or more of the following conditions:
(1) in step S10, the solvent is one or more of an organic acid solvent, a chlorinated alkane solvent, and water, for example, a mixed solvent of an organic acid solvent, a chlorinated alkane solvent, and water;
the chlorinated alkane solvent may be one or more of dichloromethane, 1,2-dichloroethane, and chloroform, for example, dichloromethane;
the organic acid solvent may be formic acid and/or acetic acid, for example, acetic acid;
in the mixed solvent, the volume ratio of the chlorinated alkane solvent to water may be (1 to 10):1, for example, 7:1;
in the mixed solvent, the volume ratio of the organic acid solvent to water may be (1 to 10):1, for example, 2:1;
(2) in step S10, the molar volume ratio of the compound of formula 6 to the solvent is (0.01 to 1) mol: 1 L, for example, (0.01 to 0.3) mol: 1 mL, for another example, 0.13 mol: 1 L;
(3) the oxidant is one or more selected from hydrogen peroxide, meta-chloroperoxybenzoic acid, and magnesium monoperoxyphthalate hexahydrate, for example, magnesium monoperoxyphthalate hexahydrate;
(4) in step S10, the molar ratio of the oxidant to the compound of formula 6 is (1 to 5):1, for example, (1.1 to 2):1, for another example, 1.5:1;
(5) in step S10, the temperature of the oxidation reaction is -20°C to 25°C, for example, 0°C to 20°C, for another example, 15°C;
(6) in step S10, the oxidation reaction further comprises the following step of mixing the compound of formula 6 with the solvent, and adding the oxidant; the oxidant may be added at a temperature of -5 to 5°C, for example, at 0°C;
(7) in step S10, the duration of the oxidation reaction is 1 to 5 hours, for example, 3 hours; and
(8) in step S10, after completion of the oxidation reaction, one or more of the following work-up steps are comprised: quenching, extraction, drying, concentration, and purification; the solvent for said quenching may be an aqueous solution of sodium thiosulfate; the solvent for said extraction may be dichloromethane; the desiccant for said drying may be anhydrous sodium sulfate; said concentration may be distillation under reduced pressure; said purification may be column chromatography, where the eluent for said column chromatography may be a mixed solvent of dichloromethane and ethyl acetate, for example, a mixed solvent with a volume ratio of dichloromethane to ethyl acetate of 4:1.

7. The preparation method of the compound of formula A8 according to claim 5, wherein the preparation method of the compound of formula 7 further comprises a preparation method of the compound of formula 6, specifically comprising the following step S9: performing a cyclization reaction as shown below on a compound of formula 6-2 in a solvent in the presence of a cyclization agent to obtain the compound of formula 6,

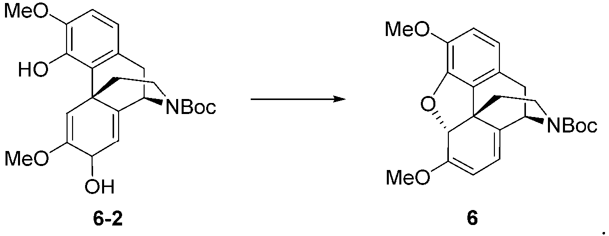

8. The preparation method of the compound of formula A8 according to claim 7, wherein the preparation method satisfies one or more of the following conditions:
(1) in step S9, the cyclization reaction is performed under the atmosphere of an inert gas, which may be nitrogen and/or argon, for example, nitrogen;
(2) in step S9, the solvent is a chlorinated alkane solvent, which may be one or more of dichloromethane, 1,2-dichloroethane, and chloroform, for example, dichloromethane;
(3) in step S9, the molar volume ratio of the compound of formula 6-2 to the solvent is (0.1 to 2) mol: 1 L, for example, 0.9 mol: 1 L;
(4) in step S9, the cyclization agent is one or more of N,N-dimethylformamide dimethyl acetal, p-toluic acid, sodium methoxide, and sodium ethoxide, for example, N,N-dimethylformamide dimethyl acetal;
(5) in step S9, the molar ratio of the cyclization agent to the compound of formula 6-2 is (1 to 5):1, for example, (1 to 3):1, for another example, 1.5:1;
(6) in step S9, the temperature of the cyclization reaction is 20 to 35°C, for example, 25°C;
(7) in step S9, the duration of the cyclization reaction is 1 to 5 hours, for example, 3 hours; and
(8) in step S9, after completion of the cyclization reaction, one or more of the following work-up steps are comprised: extraction, drying, concentration, and purification; the solvent for said extraction may be a mixed solvent of petroleum ether and ethyl acetate, for example, a mixed solvent with a volume ratio of petroleum ether to ethyl acetate of 6:1; the desiccant for said drying may be anhydrous sodium sulfate; said concentration may be distillation under reduced pressure; said purification may be crystalization; the solvent for said crystalization may be a mixed solvent of methanol and water, for example, a mixed solvent with a volume ratio of methanol to water of 4:1; the temperature of said crystallization may be -20 to 0°C, for example, -10°C.

9. A preparation method of the compound of formula A8 according to claim 7, wherein the preparation method of the compound of formula 6 further comprises a preparation method of the compound of formula 6-2, specifically comprising the following step S8: performing a reduction reaction as shown below on a compound of formula 6-1 in a solvent in the presence of a reducing agent to obtain the compound of formula 6-2,

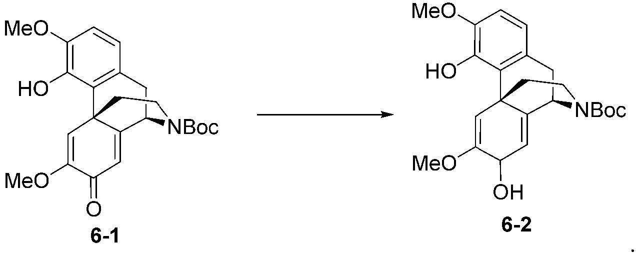

10. The preparation method of the compound of formula A8 according to claim 9, wherein the preparation method satisfies one or more of the following conditions:
(1) in step S8, the reduction reaction is performed under the atmosphere of an inert gas, which may be nitrogen and/or argon, for example, nitrogen;
(2) in step S8, the solvent is an ether solvent, for example, tetrahydrofuran;
(3) in step S8, the molar volume ratio of the compound of formula 6-1 to the solvent is (0.1 to 2) mol: 1 L, for example, 0.22 mol: 1 L;
(4) in step S8, the reducing agent is one or more selected from alkali metal borohydrides, lithium aluminum hydride, and diisobutylaluminum hydride, for example, diisobutylaluminum hydride, for another example, a solution of diisobutylaluminum hydride in tetrahydrofuran;
(5) in step S8, the molar ratio of the reducing agent to the compound of formula 6-1 is (1 to 5):1, for example, (1 to 3):1, for another example, 2: 1;
(6) in step S8, the temperature of the reduction reaction is -5 to 35°C, for example, 10 to 30°C, for another example, 15°C;
(7) in step S8, the duration of the reduction reaction is 0.1 to 2 hours, for example, 0.5 hours;
(8) step S8 further comprises the following steps: mixing the compound of formula 6-1 with the solvent, followed by adding the reducing agent to perform the reduction reaction; the reducing agent may be added at a temperature of -25 to 5°C, for example, at -20°C or 5°C; and
(9) in step S8, after completion of the reduction reaction, one or more of the following work-up steps are comprised: quenching, extraction, drying, and concentration; the solvent for said quenching may be an aqueous solution of sodium hydroxide, for example, an aqueous solution of sodium hydroxide with a molar concentration of 4 N; the solvent for said extraction may be dichloromethane; the desiccant for said drying may be anhydrous sodium sulfate; and said concentration may be distillation under reduced pressure.

11. The preparation method of the compound of formula A8 according to claim 9, wherein the preparation method of the compound of formula 6-2 further comprises a preparation method of the compound of formula 6-1, specifically comprising the following step S7: performing a debenzylation reaction as shown below on a compound of formula 5 in a solvent in the presence of a hydrogen source and a palladium catalyst to obtain the compound of formula 6-1,

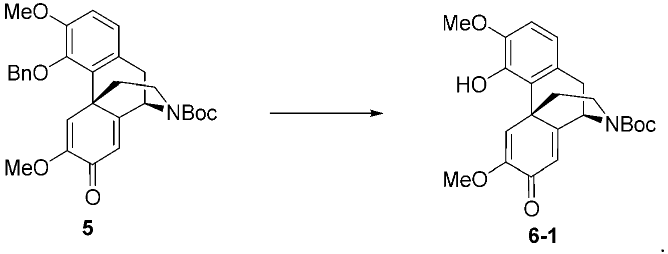

12. The preparation method of the compound of formula A8 according to claim 11, wherein the preparation method satisfies one or more of the following conditions:
(1) in step S7, the debenzylation reaction is performed under the atmosphere of an inert gas, which may be nitrogen and/or argon, for example, nitrogen;
(2) in step S7, the solvent is an ether solvent or an amide solvent, for example, an amide solvent, for another example, N,N-dimethylacetamide;
(3) in step S7, the molar volume ratio of the compound of formula 5 to the solvent is (0.1 to 1) mol: 1 L, for example, 0.4 mol: 1 L;
(4) in step S7, the hydrogen source is hydrogen and/or sodium hydride, for example, sodium hydride;
(5) in step S7, the molar ratio of the hydrogen source to the compound of formula 5 is (1 to 2):1, for example, 1.5:1;
(6) in step S7, the palladium catalyst is one or more selected from dry palladium on carbon, palladium hydroxide, palladium chloride, and palladium acetate, for example, palladium chloride;
(7) in step S7, the molar ratio of the palladium catalyst to the compound of formula 5 is (0.01 to 0.5):1, for example, 0.05:1;
(8) in step S7, the temperature of the debenzylation reaction is 10 to 30°C, for example, 25°C;
(9) in step S7, the duration of the debenzylation reaction is 1 to 20 hours, for example, 10 hours; and
(10) in step S7, after completion of the debenzylation reaction, one or more of the following work-up steps are comprised: quenching, extraction, drying, and concentration; the solvent for said quenching may be water; the solvent for said extraction may be ethyl acetate; the desiccant for said drying may be anhydrous sodium sulfate; and said concentration may be distillation under reduced pressure.

13. The preparation method of the compound of formula A8 according to claim 11, wherein the preparation method of the compound of formula 6-1 further comprises a preparation method of the compound of formula 5, specifically comprising the following step S6: performing a dearomative cyclization reaction as shown below on a compound of formula 4 in a solvent under the action of a palladium catalyst, a phosphine ligand, and a base to obtain the compound of formula 5,

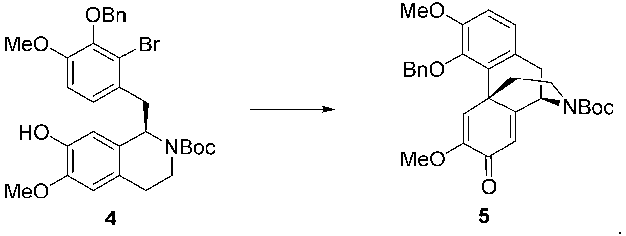

14. The preparation method of the compound of formula A8 according to claim 13, wherein the preparation method satisfies one or more of the following conditions:
(1) in step S6, the dearomative cyclization reaction is performed under the atmosphere of an inert gas, which may be nitrogen and/or argon, for example, nitrogen;
(2) in step S6, the solvent is a sulfoxide solvent and/or an amide solvent, for example, an amide solvent; the amide solvent may be one or more of N-pyrrolidone, N,N-dimethylformamide (DMF), and N,N-dimethylacetamide (DMA), for another example, N,N-dimethylacetamide;
(3) in step S6, the molar volume ratio of the compound of formula 4 to the solvent is (0.01 to 1) mol: 1 L, for example, 0.29 mol: 1 L;
(4) in step S6, the palladium catalyst is one or more of palladium chloride, bis(acetonitrile)palladium dichloride, palladium trifluoromethanesulfonate, and palladium acetate, for example, palladium chloride;
(5) in step S6, the molar ratio of the palladium catalyst to the compound of formula 4 is (0.01 to 0.5):1, for example, 0.04: 1;
(6) in step S6, the phosphine ligand is one or more of tri-tert-butylphosphine, tricyclohexylphosphine, di(1-adamantyl)-n-butylphosphine, diadamantylisopropylphosphine, and diadamantylpropylphosphine, for example, di(1-adamantyl)-n-butylphosphine;
(7) in step S6, the molar ratio of the phosphine ligand to the compound of formula 4 is (0.01 to 0.5):1, for example, 0.06: 1;
(8) in step S6, the base is an alkali metal carbonate, for example, potassium carbonate or potassium phosphate;
(9) in step S6, the molar ratio of the base to the compound of formula 4 is (1 to 5):1, for example, 2:1;
(10) in step S6, the temperature of the dearomative cyclization reaction is 100°C to 180°C, for example, 140°C;
(11) in step S6, the duration of the dearomative cyclization reaction is 3 to 10 hours, for example, 6 hours; and
(12) in step S6, after completion of the dearomative cyclization reaction, one or more of the following work-up steps are comprised: filtration, concentration, drying, and purification; said concentration may be distillation under reduced pressure; said purification may be column chromatography; the eluent for said column chromatography may be a mixed solvent of dichloromethane and methanol, for example, a mixed solvent with a volume ratio of dichloromethane to methanol of (150 to 100): 1.

15. The preparation method of the compound of formula A8 according to claim 13, wherein the preparation method of the compound of formula 5 further comprises a preparation method of the compound of formula 4, specifically comprising the following step S5: performing a reaction as shown below on a compound of formula 4-3' with di-tert-butyl dicarbonate in a solvent to obtain the compound of formula 4,

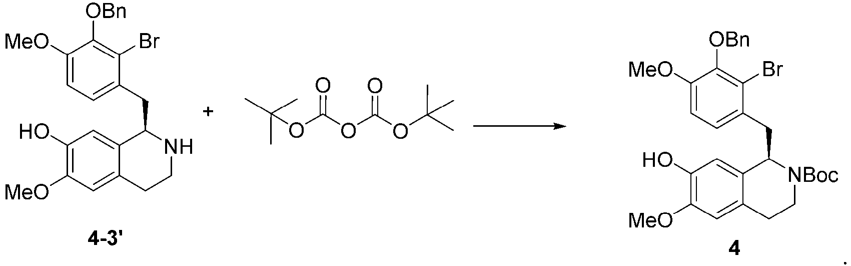

16. The preparation method of the compound of formula A8 according to claim 15, wherein the preparation method satisfies one or more of the following conditions:
(1) in step S5, the solvent is a chlorinated alkane solvent, which may be one or more of dichloromethane, 1,2-dichloroethane, and chloroform, for example, dichloromethane;
(2) in step S5, the molar volume ratio of the compound of formula 4-3' to the solvent is (0.01 to 1) mol: 1 L, for example, 0.09 mol: 1 L;
(3) in step S5, the molar ratio of di-tert-butyl dicarbonate to the compound of formula 4-3' is (1 to 3):1, for example, 1.08:1;
(4) in step S5, the temperature of the reaction is 10 to 30°C, for example, 25°C;
(5) in step S5, the duration of the reaction is 1 to 5 hours, for example, 3 hours;
(6) in step S5, after completion of the reaction, one or more of the following work-up steps are further comprised: concentration, crystallization, filtration, and drying; said concentration may be distillation under reduced pressure; the solvent for said crystallization may be methanol; said drying may be oven drying.

17. The preparation method of the compound of formula A8 according to claim 15, wherein the preparation method of the compound of formula 4 further comprises a preparation method of the compound of formula 4-3', specifically comprising the following step S4':
performing a reaction as shown below on a compound of formula 4-3 with a base in a solvent
to obtain the compound of formula 4-3',

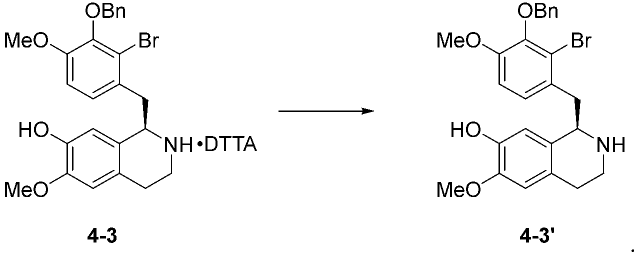

18. The preparation method of the compound of formula A8 according to claim 17, wherein the preparation method satisfies one or more of the following conditions:
(1) in step S4', the solvent is a chlorinated hydrocarbon solvent, wherein the chlorinated alkane solvent may be one or more of dichloromethane, 1,2-dichloroethane, and chloroform, for example, dichloromethane;
(2) in step S4', the molar volume ratio of the compound of formula 4-3 to the solvent is (0.01 to 1) mol: 1 L, for example, 0.13 mol: 1 L;
(3) in step S4', the base is an inorganic base, for example, potassium bicarbonate, for another example, an aqueous solution of potassium bicarbonate;
(4) in step S4', the molar ratio of the base to the compound of formula 4-3 is (1 to 10):1, for example, (4 to 6):1, for another example, 5:1;
(5) in step S4', the temperature of the reaction is 20 to 30°C, for example, 25°C;
(6) in step S4', the duration of the reaction is 0.3 to 12 hours, for example, 1 hour; and
(7) in step S4', after the completion of the reaction, the following work-up step is further comprised: extraction; the solvent for said extraction may be dichloromethane.

19. The preparation method of the compound of formula A8 according to claim 17, wherein the preparation method of the compound of formula 4-3' further comprises a preparation method of the compound of formula 4-3, specifically comprising the following step S4: performing a reaction as shown below on a compound of formula 4-2 with D-(+)-di-p-toluoyl tartaric acid in a solvent to obtain the compound of formula 4-3,

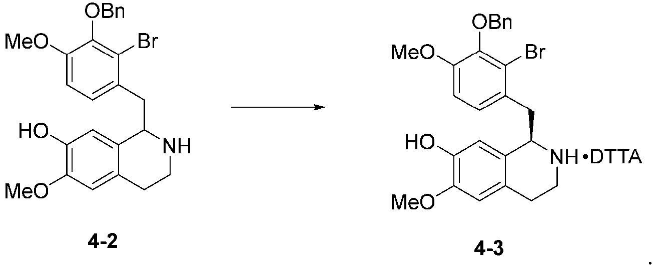

20. The preparation method of the compound of formula A8 according to claim 19, wherein the preparation method satisfies one or more of the following conditions:
(1) in step S4, the reaction is performed in an open system, which may be an air system;
(2) in step S4, the solvent is an alcohol solvent, for example, one or more selected from methanol, ethanol, and isopropanol, for another example, methanol;
(3) in step S4, the molar volume ratio of the compound of formula 4-2 to the solvent is (0.1 to 1) mol: 1 L, for example, 0.62 mol: 1 L or 0.6 mol: 1 L;
(4) in step S4, the molar ratio of D-(+)-di-p-toluoyl tartaric acid to the compound of formula 4-2 is (1 to 3):1, for example, (0.9 to 1.5): 1, for another example, 1.05:1 or 1.07:1;
(5) in step S4, the temperature of the reaction is 50 to 100°C, for example, 70°C to 100°C, for another example, 85°C;
(6) in step S4, the duration of the reaction is 3 to 12 hours, for example, 8 hours;
(7) step S4 further comprises the following steps: mixing the compound of formula 4-2 with the solvent, and subsequently adding D-(+)-di-p-toluoyl tartaric acid; and
(8) in step S4, after completion of the reaction, the following work-up step is further comprised: filtration.

21. The preparation method of the compound of formula A8 according to claim 19, wherein the preparation method of the compound of formula 4-3 further comprises a preparation method of the compound of formula 4-2, specifically comprising the following step S3: performing a deprotection reaction of carbamate groups as shown below on a compound of formula 4-1 in a solvent in the presence of an organic base to obtain the compound of formula 4-2; 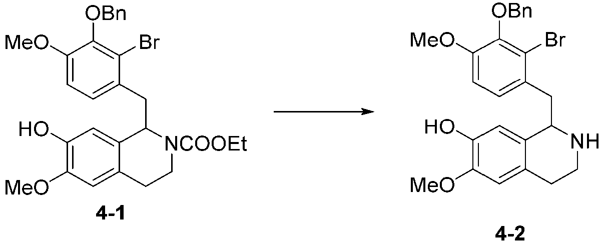 the organic base is potassium alkoxide and/or sodium alkoxide.

22. The preparation method of the compound of formula A8 according to claim 21, wherein the preparation method satisfies one or more of the following conditions:
(1) in step S3, the solvent is an ether solvent and/or a chlorinated hydrocarbon solvent, for example, an ether solvent; the ether solvent may be one or more of tetrahydrofuran, 1,4-dioxane, diethyl ether, and methyl tert-butyl ether, for another example, tetrahydrofuran;
(2) in step S3, the molar volume ratio of the compound of formula 4-1 to the solvent is (0.01 to 3) mol: 1 L, for example, (0.1-1) mol: 1 L, for another example, 0.31 mol: 1 L or 0.35 mol: 1 L;
(3) in step S3, the potassium alkoxide is potassium methoxide, potassium ethoxide, or potassium tert-butoxide, for example, potassium tert-butoxide;
(4) in step S3, the sodium alkoxide is sodium methoxide, sodium ethoxide, or sodium tert-butoxide;
(5) in step S3, the reaction further comprises an inorganic base in its system; the inorganic base may be potassium hydroxide and/or sodium hydroxide, for example, potassium hydroxide;
the molar ratio of the organic base to the inorganic base may be (0.5 to 2):1, for example, 1:1;
(6) in step S3, the molar ratio of the organic base to the compound of formula 4-1 is (0.05 to 5):1, for example, (1 to 3):1, for another example, 2:1 or 1.77:1;
(7) in step S3, the temperature of the deprotection reaction of carbamate groups is 60 to 80°C, for example, 70°C;
(8) step S3 further comprises the following steps: mixing the compound of formula 4-1 with the solvent, and subsequently adding the organic base;
(9) in step S3, the duration of the deprotection reaction of carbamate groups is 5 to 24 hours, for example, 8 hours; and
(10) in step S3, after completion of the deprotection reaction of carbamate groups, one or more of the following work-up steps are further comprised: quenching, extraction, and concentration; the solvent for said quenching may be water; the solvent for said extraction may be dichloromethane; said concentration may be concentration under reduced pressure.

23. The preparation method of the compound of formula A8 according to claim 21, wherein the organic base is sodium alkoxide, for example, sodium tert-butoxide.

24. The preparation method of the compound of formula A8 according to claim 21, wherein the preparation method of the compound of formula 4-2 further comprises a preparation method of the compound of formula 4-1, specifically comprising the following step S2: performing a reaction as shown below on a compound of formula 3' in a solvent in the presence of a base to obtain the compound of formula 4-1,

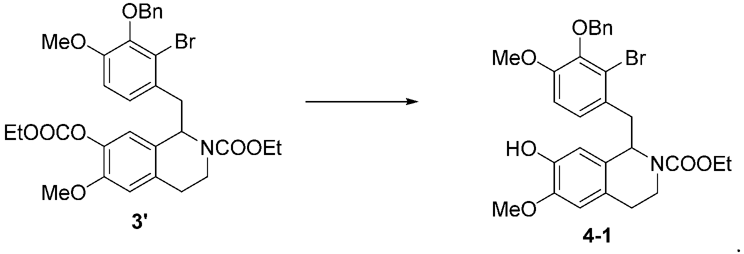

25. The preparation method of the compound of formula A8 according to claim 24, wherein the preparation method satisfies one or more of the following conditions:
(1) in step S2, the solvent is an alcohol solvent, for example, methanol and/or ethanol, for another example, ethanol;
(2) in step S2, the molar volume ratio of the compound of formula 3 to the solvent is (0.1 to 1) mol: 1 L, for example, 0.62 mol: 1 L;
(3) in step S2, the base is an inorganic base, for example, potassium hydroxide;
(4) in step S2, the molar ratio of the base to the compound of formula 3 is (1 to 3):1, for example, 2:1;
(5) in step S2, the temperature of the reaction is 30°C to 70°C, for example, 50°C;
(6) in step S2, the duration of the reaction is 1 to 10 hours, for example, 3 hours; and
(7) in step S2, after completion of the reaction, one or more of the following work-up steps are further comprised: quenching, slurrying, and drying; the solvent for said quenching may be water; said drying may be oven drying; the solvent for said slurrying may be methanol.

26. The preparation method of the compound of formula A8 according to claim 24, wherein the preparation method of the compound of formula 4-1 further comprises a preparation method of the compound of formula 3', specifically comprising the following step S2': performing a reaction as shown below on a compound of formula 3 with benzyl bromide in a solvent in the presence of a base to obtain the compound of formula 3',

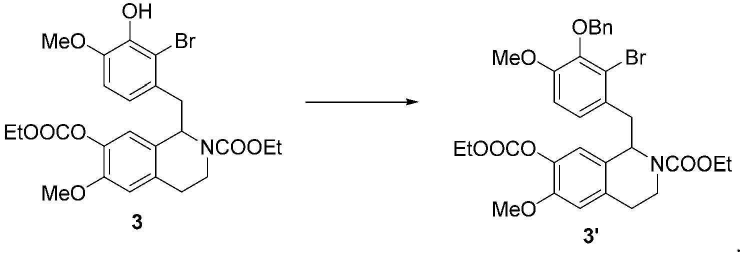

27. The preparation method of the compound of formula A8 according to claim 26, wherein the preparation method satisfies one or more of the following conditions:
(1) in step S2', the reaction is performed under the atmosphere of an inert gas, which may be nitrogen and/or argon, for example, nitrogen;
(2) in step S2', the solvent is an amide solvent, for example, N,N-dimethylformamide (DMF) and/or N,N-dimethylacetamide (DMA), for another example, N,N-dimethylformamide;
(3) in step S2', the molar volume ratio of the compound of formula 3 to the solvent is (0.1 to 1) mol: 1 L, for example, 0.62 mol: 1 L;
(4) in step S2', the base is an alkali metal carbonate, for example, potassium carbonate;
(5) in step S2', the molar ratio of the base to the compound of formula 3 is (1 to 3):1, for example, 2:1;
(6) in step S2', the molar ratio of the benzyl bromide to the compound of formula 3 is (1 to 3):1, for example, 2:1;
(7) in step S2', the temperature of the reaction is 10°C to 30°C, for example, 25°C;
(8) in step S2', the duration of the reaction is 3 to 10 hours, for example, 6 hours; and
(9) in step S2', after the completion of the reaction, the following work-up step is further comprised: crystallization; the solvent for said crystallization may be water.

28. The preparation method of the compound of formula A8 according to claim 26, wherein the preparation method of the compound of formula 3' further comprises a preparation method of the compound of formula 3, specifically comprising the following step S1: performing a condensation cyclization reaction as shown below on a compound of formula 1 and a compound of formula 2 in a solvent in the presence of a acid to obtain the compound of formula 3; 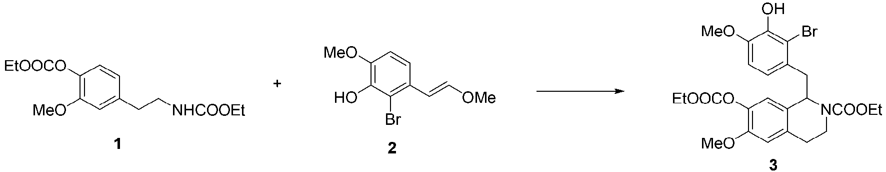 the acid is one or more of methanesulfonic acid, trifluoroacetic acid, p-toluenesulfonic acid, and p-toluenesulfonic acid monohydrate.

29. The preparation method of the compound of formula A8 according to claim 28, wherein the preparation method satisfies one or more of the following conditions:
(1) in step S1, the condensation cyclization reaction is performed in an open system, which may be an air system;
(2) in step S1, the solvent is a chlorinated alkane and/or an amide, for example, a chlorinated alkane organic solvent; the chlorinated alkane may be one or more of dichloromethane, 1,2-dichloroethane, and chloroform, for another example, dichloromethane;
(3) in step S1, the molar volume ratio of the compound of formula 1 to the solvent is (0.1 to 5) mol: 1 L, for example, (0.1 to 2) mol: 1 L, for another example, 0.78 mol: 1 L;
(4) in step S1, the molar ratio of the acid to the compound of formula 1 is (1 to 5):1, for example, (1 to 3):1, for another example, 2:1;
(5) in step S1, the acid is p-toluenesulfonic acid and/or p-toluenesulfonic acid monohydrate, for example, p-toluenesulfonic acid monohydrate;
(6) in step S1, the molar ratio of the compound of formula 2 to the compound of formula 1 is (1 to 1.5):1, for example, 1.4:1;
(7) in step S1, the temperature of the condensation cyclization reaction is 0 to 30°C, for example, 25°C;
(8) in step S1, the duration of the condensation cyclization reaction is 2 to 24 hours, for example, 16 hours; and
(9) in step S1, after completion of the reaction, one or more of the following work-up steps are further comprised: quenching, drying, concentration, and purification; the solvent for said quenching may be water; the desiccant for said drying may be anhydrous sodium sulfate; said concentration may be distillation under reduced pressure; said purification may be recrystallization, and the solvent for said recrystallization may be a mixed solvent of ethyl acetate and petroleum ether, for example, a mixed solvent with a volume ratio of ethyl acetate to petroleum ether of 2:1.

30. The preparation method of the compound of formula A8 according to claim 28, wherein the preparation method of the compound of formula 3 further comprises a preparation method of the compound of formula 1, specifically comprising the following step S1-1: performing a reaction as shown below on a compound of formula 1-2 with ethyl chloroformate in a solvent to obtain the compound of formula 1,

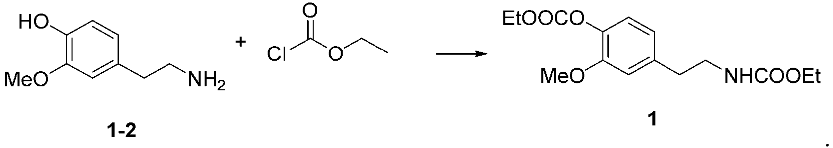

31. The preparation method of the compound of formula A8 according to claim 30, wherein the preparation method satisfies one or more of the following conditions:
(1) in step S1-1, the solvent is an ether solvent, for example, tetrahydrofuran;
(2) in step S1-1, the molar volume ratio of the compound of formula 1-2 to the solvent is (0.1 to 5) mol: 1 L, for example, (0.1 to 1) mol: 1 L, for another example, 0.1 mol: 1 L;
(3) in step S1-1, the molar ratio of ethyl chloroformate to the compound of formula 1-2 is (1 to 5):1, for example, (1 to 3):1, for another example, 2.2:1;
(4) in step S1-1, the temperature of the reaction is 10 to 30°C, for example, 25°C;
(5) in step S1-1, the duration of the reaction is 1 to 30 hours, for example, 16 hours; and
(6) in step S1-1, after completion of the reaction, one or more of the following work-up steps are further comprised: quenching, extraction, drying, and concentration; the solvent for said quenching may be water; the desiccant for said drying may be anhydrous sodium sulfate; said concentration may be distillation under reduced pressure; said purification may be recrystallization, and the solvent for said extraction may be ethyl acetate.

32. A preparation method of a compound of formula A9, comprising the following step S14: performing a demethylation reaction as shown below on a compound of formula A8 in a solvent in the presence of a demethylating agent to obtain a compound of formula A9; 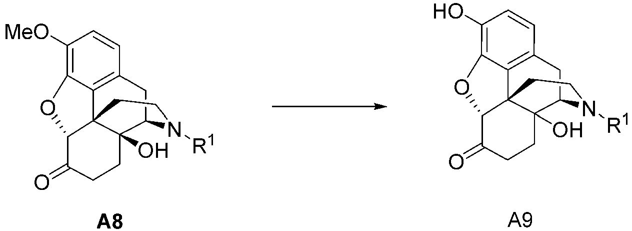 the preparation method of the compound of formula A9 further comprises a preparation method of the compound of formula A8, specifically comprising the following steps S11, S12, and S13:
S11: performing a hydrogenation reaction as shown below on a compound of formula 7 with hydrogen in a solvent in the presence of a catalyst to obtain a compound of formula 8-1; 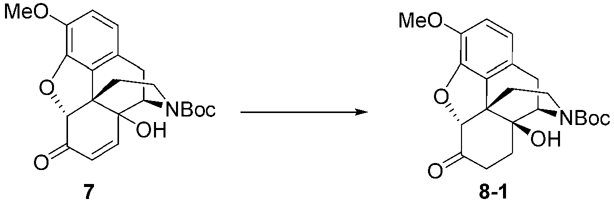
S12: performing a deprotection reaction as shown below on the compound of formula 8-1 in a solvent in the presence of a deprotecting reagent to obtain a compound of formula 8-2; 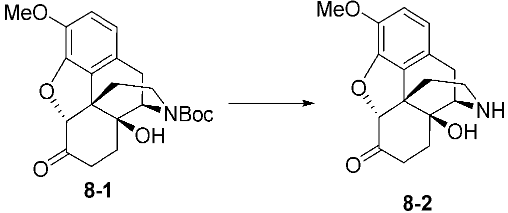
S13: performing a substitution reaction as shown below on the compound of formula 8-2 with a compound of formula SM1 in a solvent in the presence of a base to obtain a compound of formula A8; 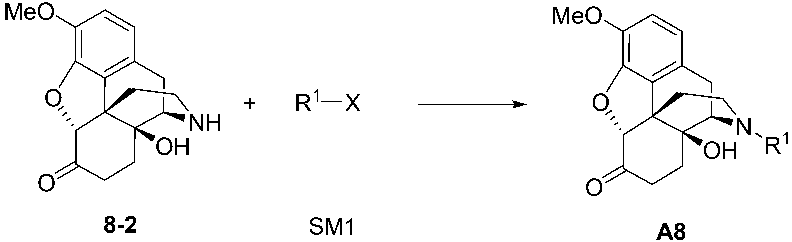
wherein X and R¹ are as defined in any one of claims 1 to 31;
the preparation method of the compound of formula A8 may be as described in any one of claims 1 to 31.

33. The preparation method of the compound of formula A9 according to claim 32, wherein the preparation method satisfies one or more of the following conditions:
(1) in step S14, the compound of formula A8 is 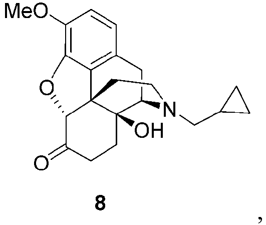 and the compound of formula A9 is 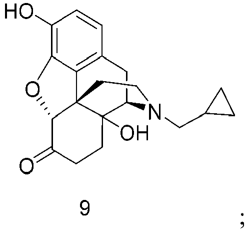
(2) in step S14, the solvent is a chlorinated alkane, which may be one or more of dichloromethane, 1,2-dichloroethane, and chloroform, for example, dichloromethane;
(3) in step S14, the molar volume ratio of the compound of formula A8 to the solvent is (0.01 to 0.1) mol: 1 L, for example, 0.045 mol: 1 L;
(4) in step S14, the demethylation reaction is performed under the atmosphere of an inert gas, which may be nitrogen and/or argon, for example, nitrogen;
(5) in step S14, the demethylating agent is boron trichloride and/or boron tribromide, for example, boron tribromide;
(6) in step S14, the molar ratio of the demethylating agent to the compound of formula A8 is (5 to 10):1, for example, (6 to 8):1, for another example, 7:1;
(7) in step S14, the temperature of the demethylation reaction is -20 to 25°C, for example, 10°C;
(8) in step S14, the duration of the demethylation reaction is 1 to 5 hours, for example, 2 hours; and
(9) in step S14, after completion of the demethylation reaction, one or more of the following work-up steps are comprised: quenching, pH adjustment, extraction, drying, concentration, and purification; the solvent for said quenching may be a saturated aqueous solution of ammonium chloride; the solvent for said pH adjustment may be a saturated 10% NaHCO₃ aqueous solution; the solvent for said extraction may be dichloromethane; the desiccant for said drying may be anhydrous sodium sulfate; said concentration may be distillation under reduced pressure; said purification may be column chromatography; the eluent for said column chromatography may be a mixed solvent of dichloromethane and methanol, for example, a mixed solvent with a volume ratio of dichloromethane to methanol of 50:1.

34. A preparation method of a compound of formula 4-3', comprising the following step S4': performing a reaction as shown below on a compound of formula 4-3 with a base in a solvent to obtain the compound of formula 4-3', 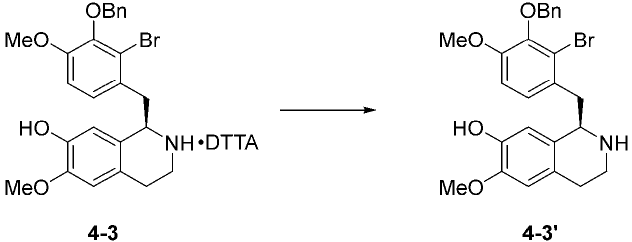 the conditions and operations of step S4' may be as described in claim 18, and the preparation method of the compound of formula 4-3' may be as described in any one of claims 19 to 31.

35. A preparation method of a compound of formula 4-2, comprising the following step S3: performing a reaction as shown below on a compound of formula 4-1 in a solvent in the presence of an organic base to obtain the compound of formula 4-2; 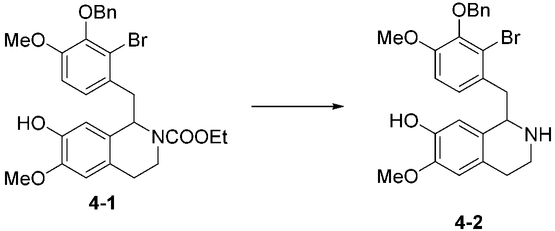
the organic base is potassium alkoxide and/or sodium alkoxide;
the conditions and operations of step S3 may be as described in claim 22 or 23; the preparation method of the compound of formula 4-2 may be as described in any one of claims 24 to 31.

36. A preparation method of a compound of formula 3, comprising the following step S1: performing a reaction as shown below on a compound of formula 1 and a compound of formula 2 in a solvent in the presence of an acid to obtain the compound of formula 3, 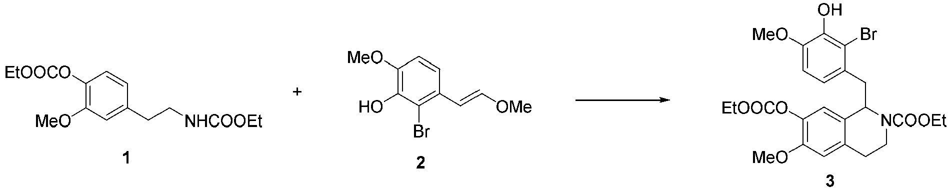
the acid is one or more of methanesulfonic acid, trifluoroacetic acid, p-toluenesulfonic acid, and p-toluenesulfonic acid monohydrate;
the conditions and operations of step S1 may be as described in claim 29; the preparation method of the compound of formula 3 may be as described in claim 30 or 31.

37. A compound of formula 7, 6-2, 5, 4, 4-3, 4-1, 3, or 1,
